# EUROPEAN PATENT APPLICATION

(11) **EP 4 104 863 A1**
(43) Date of publication of application: **21.12.2022**
(21) Application number: 21754359.4
(22) Date of filing: 12.02.2021
(51) Int. Cl.: A61K 47/00, A61K 47/16, A61K 38/00, A61L 31/06, A61L 31/16

(54) **METHOD FOR PRODUCING DECELLULARIZED BIOMATERIAL, DECELLULARIZED BIOMATERIAL AND USE THEREOF**

(30) Priority: 14.02.2020 US 202062976956 P
(71) Applicant: KHEIROS PATER INOVAÇÃO S.A., 38411-159 Uberlândia - MG (BR); Universidade Federal de Uberlândia, MG 38400-902 Uberlândia (BR)
(72) Inventor: RICARDO GOULART FILHO, Luiz, 38400-652 Uberlândia - MG (BR); DE SOUZA CASTRO FILICE, Léticia, 38408-444 Uberlândia - MG (BR); PEIXOTO RODRIGUES, Jessica, 38411-159 Uberlândia - MG (BR); REGINA DA COSTA SILVA, Jéssica, 38415-264 Uberlândia - MG (BR); BATISTA FERNANDES GASPARI, Paula, 38400-460 Uberlândia - MG (BR); CAROLINE RESENDE CORRÊA, Natássia, 38408-056 Uberlândia (BR); REZENDE ROSA, Roberta, 38.400- 718 Uberlândia - MG (BR); MENDONÇA RODRIGUES, Cláudia, 38400-654 Uberlândia - MG (BR); CARVALHO BARBOSA, Matheus, 38400-656 Uberlândia - MG (BR); SANTOS PIMENTEL, Leticia, 38405-329 Uberlândia - MG (BR); SOUSA QUIRINO, Ludmilla, 38414200 Uberlândia - MG (BR)
(74) Representative: Potter Clarkson
(86) International application number: PCT/BR2021/050069
(87) International publication number: WO 2021/159198

(57) **Abstract**

This patent application refers to the field of tissue engineering. It particularly refers to a method of producing decellularized biomaterial from biological tissue, as well as to the decellularized biomaterial produced by said method comprising biological extracellular matrix. Particularly, said biological tissue derives from animal or human ocular connective tissue. More particularly, it refers to a method of producing decellularized biomaterial from animal sclera for producing a biological scaffold, prosthesis and/or carrier of active substances (or combined with active substances), as well as biomaterial itself and uses thereof as scaffolds, prosthesis or carriers of substances for systemic or localized release.

## Description

### Field of the invention

This patent application refers to the field of tissue engineering. It particularly refers to a method of producing decellularized biomaterial from biological tissue, as well as to the decellularized biomaterial produced by said method comprising biological extracellular matrix. Particularly, said biological tissue derives from animal or human eye's connective tissue. More particularly, it refers to a method of producing decellularized biomaterial from animal sclera for producing a biological scaffold, prostheses and/or as carriers of active substances for systemic or localized release, as well as biomaterial itself and uses thereof.

### Background of the Invention

In tissue engineering, the development of novel biomaterials is essential to increasingly improve the characteristics necessary to enhance the effectiveness of these materials when used as scaffolds or for producing prosthesis, reducing the possibility of rejection of these biomaterials in biological systems. Therefore, a biomaterial can be defined as a material intended to interface with biological systems in order to evaluate, treat, increase or replace an organ, tissue or function of the organism.

The development of regenerative medicine is based on the use of a matrix or scaffold that will function as a support framework for cells of interest in order to assist in the regeneration or construction of a new tissue, as well as allowing the transportation of nutrients, metabolites, growth factors, signaling molecules, other regulatory molecules, and medicaments, among others.

Scaffolds can be synthetic or biological. The use of synthetic scaffolds is advantageous, as their chemical and physical properties can be easily manipulated according to the objective proposed and according to the cell type to be used. However, biological scaffolds, unlike synthetic ones, are compounds of natural extracellular matrix derived from tissues, such as tendons, ligaments, submucosa of small intestine, bladder and liver (BADYLAK, 2008; MENG et al., 2014). Thus, since biological scaffolds have natural extracellular matrix, they can retain growth factors and structural elements such as elastin, fibronectin and collagen, providing a favorable environment for adherence, differentiation and cell proliferation (PARENTEAU-BAREIL et al. 2010).

Scaffolds offer structural, biochemical, and biomechanical properties capable of directing and regulating cell behavior and tissue development (GILPIN & YANG, 2017). Cells that are used in tissue engineering can be from the patient himself (autologous), from another individual (allogenic) or individuals of different (xenogenic) species (ABBAS; LICHTMAN; PILLAI, 2012).

In order to reduce the chances of immune reaction and rejection of biological scaffolds, decellularization is a widely used technique, as it eliminates the presence of possible antigenic particles (BADYLAK et al., 2002). Decellularization can be performed by chemical, enzymatic, physical, and combinative procedures. This strategy seeks to remove the cells from the tissue at issue, and eliminates the genetic material present there, to prevent possible immune reactions and maintain the elements of the extracellular matrix preserving the mechanical properties necessary to maintain scaffold functionality (GILPIN & YANG, 2017). Although decellularization is a process used, there are different methods for carrying out the same. Each method has its particularities, advantages and disadvantages, and are applied in different biological materials from different origins.

Patent CN107007886 describes decontamination of bovine or porcine intestinal submucosa using peracetic acid and ethanol and, after viral inactivation, using trypsin, PBS, EDTA and submucosa ultrasonication for decellularization. This patent does not use detergents nor nuclease and it uses an expensive equipment, which is the ultrasonicator, and it does not mention any eye tissue.

Patent CN105944142 describes three methods of decellularization of tendon or ligament, using a protective solution (cell culture medium, chondroitin, dextran, antibiotic and hyaluronic acid, under pressure) before and after treatment with detergent and enzymes. Its nuclease incubation temperature is 25 or 30°C. However, it does not use chelant (EDTA) nor hypotonic buffer, nor it uses RNase in the decellularization process, nor DNAse standard temperature, which is 37°C, and it does not mention the possibility of using it in eye tissue.

Patent CN105833353 presents a process for decellularization of porcine skin, which uses protective solution (chondroitin sulfate, hyaluronic acid, glycerol and neomycin) and a condition of high static pressure, followed by incubation with nucleases that can be performed simultaneously or separately from incubation with detergent. It does not mention the use of a chelant (EDTA) nor the use of hypotonic buffer, and it does not mention any eye tissue.

Patent CN101947144 describes a decellularized corneal scaffold. For the decellularization process, it performs freezing and thawing cycles, followed by incubation in hypotonic solution, digestion with nuclease, and electrophoresis followed by scaffold rehydration. The patent does not use sclera and predicts the use only for replacement of other corneas (transplants).

Patent EP3412322 describes methods of preparing and using membranes consisting of collagen and submitted to dehydration and decellularization, from a human placenta (amniotic and chorionic membranes). The decellularization process is based on the use of detergents, like Triton X-100 or SDS, washings preferably under vacuum, and treatment with cell adhesion factors and fetal bovine/human serum that facilitates cell culture in the biomaterial. It neither mentions the use of nucleases nor the use of EDTA, or of any eye tissue.

Patent RU2016135703 presents a decellularization process to obtain kidney matrix from rats and rabbits. The decellularization is detergent-enzymatic using a high performance and perfusion system and a bioreactor, as well as solutions (hypotonic, containing EDTA, for osmotic shock and detergents - SDS, SDC, Triton X-100). The process involves decellularization of the entire organ and it does not mention any eye tissue.

Patent application CN106730005 proposes a method for preparing a corneal stroma for decellularization, so that the collagen structure is not damaged. Although this document cites sclera, the invention presents as its product the obtainment of corneal stroma that comprises a cornea and a sclera linked to the corneal periphery, and the scleral tissue with a width of only 2-3 mm. Decellularization itself and the use of sclera are not in the focus of the invention.

Patent application US2018256784 presents several methods of bovine intervertebral disc decellularization, which uses ultrasonication with decellularization solutions (nonionic surfactant and protease inhibitor), washings with water and 70% ethanol solution and nuclease digestion for 48 hours. It also demonstrates the methods of evaluating the efficiency of decellularization as a quantification of residual DNA and glycosaminoglycans. It reports incubation with nucleases for extended time, requires the ultrasonicator and does not mention ocular tissues.

Patent application KR20180133172 presents a method for producing an injectable adhesive to fix bone grafts from a decellularized extracellular matrix. The sample tissue was the tibia, incubated for previous demineralization, followed by decellularization with trypsin, and degassed by stirring. It does not provide details of the solutions, nor it mentions any tissue of eye origin.

Patent application US201816121969 describes decellularization and delipidation of adipose tissue and various other types of soft tissues. The document suggests several formats that the decellularized tissue can assume, various types of preparation and preprocessing of material, stages of delipidation, decellularization and disinfection. However, it does not specifically mention sclera, much less the use of endonucleases.

Patent application KR20180003879 describes a decellularization process by degassing using a bioreactor. Although the title refers to the use in several organs, the results presented refer only to kidneys. The method described in that document differs from the method proposed by this patent application.

The study performed by Saldin et al. (2017) is a review on 31 types of tissues with different origins, comparing the decellularization protocol of the tissues. It presents the processing of various porcine tissues, including the cornea, but without mentioning the use of sclera. The decellularization process that uses EDTA and SDS is cited only for human tendon and does not use nucleases. Other protocols usually use trypsin, SDC (sodium deoxycholate) and Triton X-100, sometimes in combination with nuclease buffer.

The study by Singelyn et al. (2011) mentions that decellularization of myocardial tissue was performed with 1% SDS, until the matrix became white. The study does not mention the use of nuclease or EDTA for decellularization and it does not mention eye tissues.

The study performed by Seif-Naraghi et al. (2012) reports decellularization of porcine pericardium performed with SDS, ionic detergent and water washings. After decellularization, pericardium was freeze-dried and ground into powder. The study does not detail the decellularization process, nor it cites eye tissues.

The study performed by Liu et al. (2009) aims to manufacturing a three-dimensional porous scaffold of bladder submucosa and subsequent recellularization of the scaffold. Porcine bladders were washed and oxidized for decellularization by immersion in peracetic acid (at different concentrations) for 4 hours. Then, they were treated with Triton X-100 for 2 days and washed with distilled water for another 2 days. The decellularization process does not mention the use of detergents or nucleases and is carried out with the entire organ, without reference to eye tissues.

The study performed by Dahl et al. (2003) describes three methods of porcine carotid decellularization for use as scaffolds. The first method uses buffer with 1% Triton X-100, 0.02% EDTA and 0.2 mg/mL of DNase for 24 hours. The second method describes 11-hour incubation with hypotonic buffer with TRIS HCl, EDTA, PMSF (phenylmethylsulfonyl fluoride) and BHA (butylated hydroxyanisole) followed by 11-hour incubation of hypertonic buffer with TRIS HCl, NaCl, EDTA, PMSF and BHA. The third treatment comprises 11-hour incubation with a zwitterionic detergent solution (CHAPS) with NaCl, EDTA in PBS and followed by incubation with an anionic detergent solution (SDS) with NaCl and EDTA in PBS. All treatments were performed under 10% CO2 and 37°C under constant stirring and were washed with PBS for 5 minutes after decellularization. The processes do not mention the use of nucleases nor cite eye tissues.

The study by Tedder et al. (2009) reports on moderate cross-link (reticulated) collagen scaffolds for heart valve engineering. The scaffolds were prepared from decellularized pericardium and treated with PGG (penta-galloyl glucose), a collagen-binding polyphenol. For decellularization, the pericardium was washed in sterile saline solution and stored in water at 4°C overnight. Then, it was treated with SDC and Triton X-100, EDTA and NaN3 in TRIS HCl buffer (pH 7.8) with moderate stirring for 6 days at 22°C and the solution was changed after 3 days. After washing with water and 70% ethanol (to remove detergent residues), it was incubated in DNase and RNase at 37°C for 24 hours. After being washed, it was incubated with elastase for 6 days and then washed again until soluble protein levels were not detected in the BCA assay. The decellularization process is more time consuming, and it does not use SDS nor mention eye tissues.

The study performed by Visser et al. (2015) reports that the decellularization process of cartilage, meniscus and tendon tissue was performed with 1% Tris for 24 hours, 2-hour sonication and digestion using nuclease (DNase and RNase) for 72 hours at 37°C. Thus, the process described is longer than that proposed in our work, it does not use SDS and requires ultrasonication, and the study does not mention eye tissues.

The study performed by Paduano et al. (2016) describes that the decellularization of bone extracellular matrix was performed with trypsin and EDTA for 24 hours and subsequent incubation with streptomycin and penicillin for 24 hours for removing the residual cell material. Thus, the decellularization process under discussion does not use nucleases and SDS and does not involve eye tissues.

The study performed by Wang et al. (2010), aims at using decellularized porcine myocardium as a scaffold for cardiac tissue engineering. For decellularization, they used a bioreactor, SDS, trypsin, protease inhibitors, nucleases and sonication. The protocol lasts two and a half weeks, it is quite long when compared to that proposed in this work, and it does not mention eye tissues.

The study performed by Wang et al. (2015), evaluates the use of decellularized liver and kidney to obtain a matrix with stable and clinically applicable characteristics. The organs were submitted to perfusion for blood withdraw and to perform decellularization with solutions containing distilled water (1-3h), SDS, Triton X-100, PAA (peracetic acid) and Na-DOC (sodium deoxycholate) (2-18 to 24h). The protocol did not use nucleases and, unlike the proposed one, it requires equipment to carry out the perfusion, as it is made with an entire organ. The study does not cite eye tissues.

The study performed by Sawkins et al. (2013), sought to use demineralized and decellularized bone matrix and comparing it to the characteristics of different extracellular matrices. For demineralization, they used stirring with HCl solution, for 24 h. Subsequently, the samples were freeze dried. The decellularization process was based on the use of trypsin and EDTA, for 24 hours and incubation with streptomycin and penicillin for 24 hours. This decellularization does not use nucleases nor SDS and includes the use of trypsin. It does not mention any eye tissue.

The study performed by Wolf et al. (2012) presents decellularization of extracellular matrix from porcine dermis and bladder. For decellularization, the tissues were treated with solutions of trypsin, ethanol, hydrogen peroxide, TRIS-EDTA/Triton X-100 and peracetic acid for approximately 46 hours. The decellularization process is longer than the proposed in this application, and it does not use nucleases and SDS, nor eye tissues.

The study performed by Wu et al. (2015) presents decellularization of porcine meniscus with 1% SDS/PBS (72 hours, and this medium is exchanged every 24 hours), 0.1% EDTA/PBS (24h), deionized water (overnight) and freeze-drying. The decellularization process does not use nucleases and the study does not mention eye tissues.

The study performed by Ungerleider et al. (2015) describes decellularization of extracellular matrix of cardiac and skeletal muscle with 1% SDS and streptomycin/penicillin for 5 days. The decellularization process does not mention the use of EDTA or nucleases. The study does not mention any eye tissue.

The study performed by Fu et al. (2016), describes an extracellular matrix of porcine skeletal muscle. Decellularization was performed with SDS, pepsin, EDTA, Triton X-100 and sodium deoxycholate in 5 different methods, with different mixtures of these reagents. Decellularization does not cite tissues of eye origin.

The study performed by Martinello et al. (2012) presents a recellularized scaffold of cadaveric tissue in tendon lesions. The decellularization protocol uses TRIS-EDTA and protease inhibitors for 2 hours, followed by 0.1% SDS for 5 hours and incubation with DNase for 2 hours. It does not mention the use of eye tissue.

The study performed by Mohammadie et al. (2018) describes a decellularization protocol of bovine articular cartilage. The protocol is based on the use of physical (freezing in liquid nitrogen and thawing) and chemical processes (treatment with 2.5% SDS and washings with phosphate buffer). To eliminate residual SDS, the authors place the samples in a Buchner sterile filter and perform washes with 75% ethanol, distilled water and phosphate buffer. The decellularized scaffolds were then functionalized with carboxylated simple wall carbon nanotubes for use in cartilage engineering. It does not use EDTA and requires a specific apparatus for washings. The study does not mention eye tissues.

The study performed by Xu et al. (2017) describes the standardization of a decellularization protocol for porcine Achilles tendon. In the chemical methods evaluated, the authors test different combinations of concentrations of SDS and Triton X-100 for 48 and 72 hours of incubation. Then, physical methods (freezing and thawing, ultrasound, perfusion, hydrostatic washing) were tested combined with the optimal concentration of chemical reagents identified in the first study, and the chemical treatment time was limited to 48 hours. Finally, the samples were washed, digested with DNase and stored at -20°C. This study established as an optimal protocol a combination of two chemical detergents (0.5% SDS and 1% Triton X-100) with a physical method (washing under 200 mmHg hydrostatic pressure) to decellularize the Achilles tendons. The process does not mention the use of EDTA and requires special apparatus, nor it mentions eye tissues.

In the study performed by Sackett et al. (2018) the pancreatic tissue was initially decellularized using two 48-hour incubations with sodium deoxycholate/2.5 mM PBS exchange. After this incubation, the tissue was rinsed with water and washed in 1X PBS supplemented with antibiotics for 72 hours, rinsed with water every 24 hours and the replacement of in natura antibiotics was performed daily. The resulting decellularized pancreatic matrix was freeze dried and stored at -80°C for future use. Decellularization does not mention the use of SDS or EDTA and there is no mention of eye tissues.

Although the advantages that a biomaterial such as a decellularized extracellular matrix can bring compared to other biomaterials or synthetic materials in medicine are known, particularly in regenerative medicine, there is still a big concern about the use of this type of material. The antigenic potential, the possibility of carrying infectious agents, variability in relation to the prepared material and the lack of ability to specify and/or characterize undesirable bioactive components in said materials are important issues that increasingly require effective technical solutions. Several tissue treatment methods are currently known, and some of these methods include decellularization of these tissues and are described in the documents mentioned in this application, since they define the general state of the art.

The methods known for obtaining biomaterials for use, such as biological scaffolds, comprise time-consuming treatments, they do not have an appropriate degree of decellularization and/or they are not reproductible when applied on large-scale. Therefore, the field of tissue engineering seeks to develop improved biomaterials for use as a scaffold or prosthesis, as well as developing a method for producing these materials, that can ensure greater efficiency and lower variability of the final product.

### Object of the Invention

In view of the issues inherent to the state of the art, the present patent application aims at developing a method for producing decellularized biomaterial comprising biological extracellular matrix and capable of producing said decellularized extracellular matrix on a larger scale, shorter time, with a lower degree of variability and greater reproducibility of the final product.

Another object of this application is providing a biomaterial comprising the decellularized biological extracellular matrix with improved properties to be used as scaffold, in the production of prosthesis or as carriers of substances.

Another object of this application is providing a biomaterial comprising, in addition to the biological decellularized extracellular matrix with improved properties, at least one additional substance for systemic or local release.

Another object of this application is providing the use of this biomaterial in the production of a product as a scaffold, in the production of prosthesis or of a carrier of active substances for systemic or local release in an individual in need thereof, preferably for use in processes of tissue repairing and/or regeneration, such as for bone tissue, for cartilaginous tissue, for joints, for tendon, for skin, for prosthetic purposes in implant dentistry and/or bucomaxillofacial traumatology, for surgical procedures of filling and/or anatomic repair, among others.

Another object of this invention is to provide the use of this biomaterial in the production of a carrier product of water or alcohol soluble substances, nutrients, metabolites, growth factors, signaling molecules, regulatory molecules, hormones and medicines or pharmaceutically active compounds, among others, in an individual or animal in need thereof.

### Brief description of the invention

This patent application describes a method for producing decellularized biomaterial comprising biological decellularized extracellular matrix, particularly in which the biological tissue is derived from eye connective tissue, more particularly scleras from porcine, bovine, rabbit or human individuals. This method presents a better cost-benefit ratio, allowing the reduction of the processing time, with efficiency in the decellularization process, reducing variability between final products and enhancing reproducibility of the method proposed herein.

Another embodiment of this application involves the biomaterials comprising biological decellularized extracellular matrix with improved properties, as those produced by the proposed method, to be used as scaffold in the production of prosthesis or as carriers of substances for systemic or local release.

Another embodiment of this application comprises the biomaterials as produced by the proposed method, which comprise, in addition to the decellularized extracellular matrix of biological origin with improved properties, at least one additional active substance for systemic or local release. The refered substance can be selected from the group consisting of: water or alcohol soluble substances, nutrients, metabolites, growth factors, signaling molecules, regulatory molecules, hormones and medicaments, or pharmaceutically active compounds.

Another embodiment of this application is the use of this biomaterial in the production of a scaffold, in the production of prosthesis and/or in the production of a carrier of active substances for systemic or local release in an individual in need thereof, preferably for use in processes of tissue repair and/or regeneration, such as for bone tissue, for cartilaginous tissue, for joints, for skin, for prosthetic purposes in implant dentistry and/or bucomaxillofacial traumatology, for surgical procedures of filling and/or anatomic repair, among others.

### Brief description of the figures

The present invention will be better understood based on the following description, taken together with the attached figures, in which:
Figure 1A shows the image of an in natura porcine eye, as received, with its natural tissues.
Figure 1B shows a representative image of the tissue cutting, in order to clean the material, leaving only the scleral tissue.
Figure 2A shows a representative image of the clean sclera, just before the decellularization process - external view.
Figure 2B shows a representative image of the clean sclera, just before the decellularization process - internal view.
Figure 3 shows a representative image of a bioreactor used in the F protocol of decellularization.
Figure 4 shows the histological analysis of porcine control sclera (in natura) and of the biomaterial produced after the decellularization protocols, from A to F. (The arrows point to nuclei or nuclei residues).
Figure 5 shows a representative test plate demonstrating the collaboration obtained through the hydroxyproline quantification procedure.
Figure 6A shows the layout used for the disposition of the solutions analyzed in the nitrocellulose membrane for content analysis of type I collagen, type II collagen and elastin by dot blot.
Figures 6B, 6C and 6D show the dot blot performed for type I collagen, type III collagen and elastin, respectively.
Figures 7A and 7B show an image of scanning electron microscopy of in natura sclera samples. The arrow indicates the presence of cell.
Figures 8A and 8B show an image of scanning electron microscopy of dehydrated sclera samples. The arrow indicates the presence of cell.
Figures 9A and 9B show an image of scanning electron microscopy of dehydrated and rehydrated sclera samples. The arrow indicates the presence of cell.
Figures 10A and 10B show an image of scanning electron microscopy of samples of the produced decellularized biomaterial.
Figure 11A shows a scatter dot plot of the Young elasticity module (GPa) for in natura sclera samples, samples of decellularized biomaterial, samples of decellularized and dehydrated biomaterial and samples of decellularized, dehydrated and rehydrated biomaterial.
Figure 11B shows a scatter dot plot of maximum strain (GPa) for in natura sclera samples, samples of decellularized biomaterial, samples of decellularized and dehydrated biomaterial and samples of decellularized, dehydrated and rehydrated biomaterial.
Figures 12 A-D show the implantation procedure steps of the decellularized biomaterialin the subcutaneous tissue of an animal.
Figures 13-17 present representative images of histological cutting of biomaterial implants in an animal.
Figures 18 A-D show a statistical analysis of the evaluated parameters. A) acute inflammation; B) presence of fibroblasts; C) presence of vessels and D) presence of giant cells.
Figures 19 A-B present representative images of histological cutting of the implants of decellularized biomaterial in tendon lesion with 49 and 114 days respectively.
Figure 20 A presents a representative image of histological cutting of the implantation region of the decellularized biomaterial in tendon lesion.
Figure 20 B presents a representative image of a histological cutting of tissue adjacent to the injured native tendon.
Figures 20 C and D present a representative image of histological cuttings of a healthy native tendon that was removed for replacement by the implant with the decellularized biomaterial.
Figure 21 presents a representative image of histological cutting of the implantation region of the decellularized biomaterial in tendon lesion, showing the presence of blood vessels and hyperemia in the region of implant interaction in the first experimental time (49 days).
Figure 22 presents a representative image of histological section showing the loose connective tissue that covers the region of the injured tendon.
Figures 23 A and B present representative images of histological cutting of the decellularized biomaterial implant in interaction with the native tendon in the second experimental time (114 days).
Figures 24 A and B present representative images of histological cutting showing the profile of predominant cells of native tendon that infiltrate the biomaterial implant in the first experimental time (49 days) (A) and in the second experimental time (114 days) (B).
Figures 25 A-D present representative images of histological cuttings showing the implant in bone tissue of a commercially available product (bone graft granules marketed as Bio-OSS^{®}) after 49 days - control group.
Figures 26 A-D show representative images of histological cuttings showing the implant in bone tissue of a commercially available product (bone graft granules marketed as Bio-OSS^{®}) after 130 days - control group.
Figures 27 A and B present representative images of histological cutting showing the interaction of the decellularized biomaterial with native bone tissue after 49 days.
Figures 28 A-D present representative images of histological cutting showing the decellularized biomaterial implant in bone tissue showing the integration of the biomaterial with the native bone tissue after 130 days.
Figure 29 presents representative images of histological section showing the decellularized biomaterial implant combined with simvastatin in bone tissue after 49 days.
Figures 30 A and B present representative images of histological cutting showing implant integration of the decellularized biomaterial combined with simvastatin in bone tissue after 49 days.
Figures 31 A and B present representative images of histological cutting showing cellular infiltration in the decellularized biomaterial implant combined with simvastatin in bone tissue after 130 days.
Figure 32 presents a representative image of histological cutting showing the cartilage lesion that received the implant, after 30 days.
Figures 33 A-C present representative images of histological cuttings showing good integration between the biomaterial implant, the articular cartilage and the newly formed connective tissue.
Figures 34 A-F present images of filling surgical correction and anatomical correction of eyelid retention using the biomaterial.

### Detailed Description of the Invention

The present application describes a method for producing a decellularized biomaterial from biological tissue, comprising the steps of:
- collecting, processing and cleaning the biological tissue;
- subjecting the biological tissue to a decellularization process; and
- subjecting the decellularized biological tissue to a decontamination and/or sterilization process.

Said method further comprises a dehydration step, which can preferably occur after a decontamination and before the sterilization step, and a rehydration step of the decellularized biomaterial.

The biological tissue used in this method is derived from an eye's connective tissue, even more particularly scleras from porcine, bovine, rabbit or human origin.

This application also describes the decellularized biomaterial produced by said production method. Said biomaterial comprises the decellularized extracellular matrix of biological origin with improved properties, in which said matrix comprises type I and III collagen fibers and elastin.

Moreover, this application describes the use of said decellularized biomaterial produced as scaffold, prosthesis or carrier of active substances in an individual in need thereof.

### Example 1 - Collection and processing of samples from animal origin.

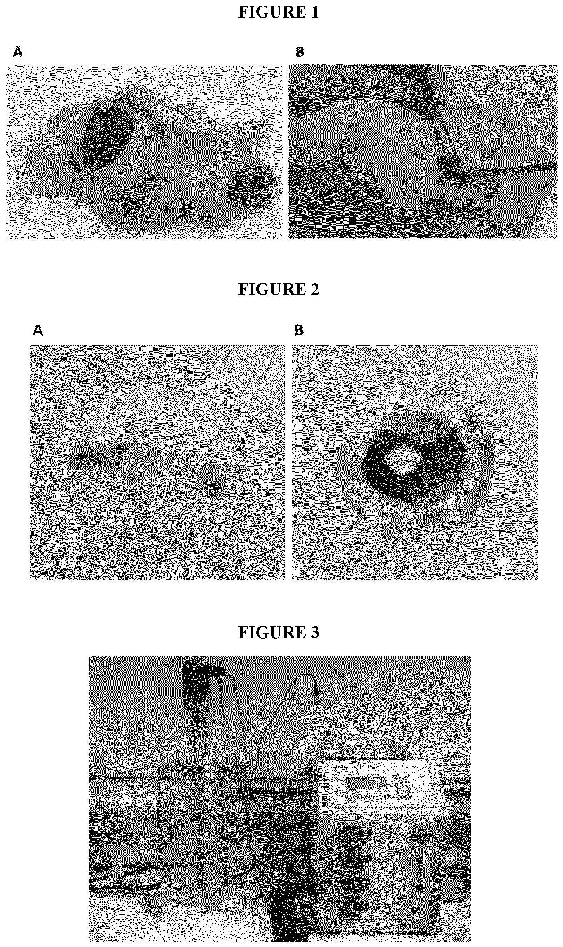

Animal eyeball samples were collected in cold storages. In the example shown herein, these are porcine eyeball samples collected in slaughterhouses that process exclusively porcines (Figure 1A). Immediately after collection, the samples were immersed in saline solution (0.9% NaCl) and kept refrigerated in a thermal container until received by the laboratory. The transport time until the laboratory should be as short as possible, and always less than 10h.

The samples were immediately processed for removing the eye components that did not correspond to the sclera, with the aid of surgical instruments (pincers, scalpel etc.) (Figure 1B).

The extracted scleras were intensely cleaned with a TBS solution (buffered TRIS saline solution) and running water (Figures 2A and 2B), for subsequent submission to the decellularization protocol.

### Example 2- Decellularization protocol of biological material.

Decellularization is a strategy that seeks reducing or eliminating the chances of adverse immunogenic reaction due to the use of biological scaffolds. This technique acts in the removal of cells from the tissue under discussion, and in the elimination of the genetic material present therein, but seeking to maintain the elements of the extracellular matrix and preserving the mechanical properties necessary for the maintenance of the scaffold functionality.

Thus, after the step of sample processing and obtaining the cleaned scleras, they were initially washed 1-5 times in a TBS solution between 1 and 20 minutes, at a temperature between 0 and 25°C, under stirring between 80 and 200 rpm. After washing, incubation was performed using a hypotonic buffer (5-20 mM TRIS, 0.05-1% w/v EDTA, pH between 6 and 8) per 1-18h, under stirring between 80-200 rpm, at a temperature between 4-37°C. After this step, the samples were treated with detergent buffer (5-20 mM TRIS, 0.05-1% w/v EDTA and 0.05-2% SDS) under stirring between 80-200 rpm per 5-24h, at a temperature between 4-37°C. Then, the samples were washed with deionized water, for 30 minutes-4 hours, at a temperature between 4-37°C, under stirring between 80-200 rpm and washed with isotonic buffer (TBS solution) for 5-15 minutes, at a temperature between 4-37°C and under stirring between 80-200 rpm. The protocol followed with incubation of biological samples with nuclease buffer (10-50 mM TRIS, 2-10 mM MgCl₂, 1-80 U/mL DNAse and 0.05-80 U/mL RNAse), for 30 minutes-3h, under stirring between 100-200 rpm, and at a temperature between 30-40°C. After this step, the samples were submitted to final washes in saline-phosphate buffer (PBS) containing 0.05-1% w/v EDTA, for up to 5 minutes at a temperature between 25-37°C, under stirring between 80-200 rpm.

The standard protocol described above was based on previous studies of decellularization, with modifications aimed to meet the particularities of the material under discussion, as well as ensuring the reproducibility of the method, time reduction and cost-benefit improvement. The work of Kasbekar et al. (2018) indicates a chemical-enzymatic decellularization process for tissues involving the use of hypotonic detergent and buffer with nucleases, which was used as an initial protocol for standardization of scleras decellularization (Table 1).

**Table 1: Description of the initial decellularization protocol and test protocols with the appropriate modifications performed.**

| INITIAL PROTOCOL | TEST PROTOCOLS | | | | | BIOREACTOR |
|---|---|---|---|---|---|---|
| Kasbekar et al., 2018 | A | B | C | D | E | F |
| 1^{st} wash with isotonic buffer (TBS) TRIS/NaCl, 20 min, 200 rpm, 25°C, 3 times | Same as Kasbekar et al., 2018 | Same as Kasbekar et al., 2018 | Same as Kasbekar et al., 2018 | Same as Kasbekar et al., 2018 | Rotation between 80-200 rpm, preferably 100 rpm | Rotation between 80-200 rpm, preferably 180 rpm |
| 2^{nd} lysis with hypotonic buffer 10 mM TRIS / 0.1% EDTA w/v, pH = 8, 18 h, 200 rpm, 4°C | Same as Kasbekar et al., 2018 | Same as Kasbekar et al., 2018 | Same as Kasbekar et al., 2018 | Same as Kasbekar et al., 2018 | Rotation between 80-200 rpm, preferably 100 rpm | Rotation between 80-200 rpm, preferably 180 rpm, preferably at 25°C |
| 3rd incubation with detergent buffer 10 mM TRIS, 0,1% EDTA w/v, 0,1% SDS w/v, 24 h, 200 rpm, 25°C | Same as Kasbekar et al., 2018 | Same as Kasbekar et al., 2018 | Same as Kasbekar et al., 2018 | Same as Kasbekar et al., 2018 | Rotation between 80-200 rpm, preferably 100 rpm | Rotation between 80-200 rpm, preferably 180 rpm |
| - | - | - | - | - | 4^{th} wash with distilled water, preferably for 4 h, 100 rpm, 25°C, with water exchange in half time | Same as Protocol E, preferably with rotation at 180 rpm, 37°C |
| 5^{th} wash with isotonic buffer (TBS) TRIS/ NaCl, 20 min, 200 rpm, 25°C, 3 times | Same as Kasbekar et al., 2018 | Same as Kasbekar et al., 2018 | Same as Kasbekar et al., 2018 | Same as Kasbekar et al., 2018 | Rotation between 80-200 rpm, preferably 100 rpm | Rotation between 80-200 rpm, preferably 180 rpm |
| 6^{th} incubation with nuclease buffer 50 mM TRIS, MgCl₂, 1 U/mL benzonase, 3 h, 150 rpm, 37°C | Same as Kasbekar et al., 2018 | Benzonase 5 U/mL (Sigma-Aldrich) | Benzonase 10 U/mL (Sigma-Aldrich) | DNase/RNase 80 U/mL (Sigma-Aldrich) | Preferably DNase/RNas e 8 U/mL (Sigma-Aldrich) | Preferably DNase 13.3 U/mL RNase 0.25 U/mL (Sigma-Aldrich) |
| 7th wash with PBS/0.1% EDTA buffer, twice | Same as Kasbekar et al., 2018 | Same as Kasbekar et al., 2018 | Same as Kasbekar et al., 2018 | Same as Kasbekar et al., 2018 | Same as Kasbekar et al., 2018 | Rotation between 80-200rpm, preferably 180 rpm, preferably at 25°C |

According to table 1, protocol A strictly followed the description in the study carried out by Kasbekar et al. (2018). The difference of the proposed protocol B is on the concentration of benzonase used in the nuclease buffer, which is raised from 1 U/mL to 5 U/mL. Protocol C, on the other hand, raises this concentration to 10 U/mL. Protocol D, by its turn, replaces benzonase with DNase and RNase (80 U/mL), while protocol E uses 8 U/mL of DNase and RNase, and reduces all rotations during the process, preferably from 80 rpm to 200 rpm, more preferably from between 90 and 180 rpm, and even more preferably 100 rpm, and add a phase comprising washings with deionized water after incubation with detergent buffer, preferably for 4 hours, at a temperature of 25°C and rotation of 100 rpm, with two exchanges in this interval. Finally, adjustments were made in protocol F in relation to the rotations, preferably from 80 rpm to 200 rpm, more preferably between 90 and 190 rpm, and even more preferably 180 rpm, temperatures, incubation times and concentrations of nucleases in order to adapt to the volume and suitable conditions to perform the process semi-automatically in the Biostat B bioreactor (B. Braun, Biotech International) (Figure 3).

According to Crapo et al. (2011), the decellularization efficiency criteria covers an number of less than 50 ng of DNA/mg of extracellular matrix (dry weight), and the lack of intact DNA and visible nuclear material in histological analysis.

According to the results obtained and described in table 2, protocols A, B and C did not reach the DNA/mg tissue limit required for efficient decellularization. These protocols used benzonase (*Serratia marcescens* nuclease), a promiscuous endonuclease that attacks and degrades all forms of DNA and RNA (Nestle and Roberts, 1969). Therefore, the combined use of nucleases (DNase/RNase) was proposed under high concentration, replacing benzonase (protocol D). This protocol was the first to meet the criteria for decellularization efficiency. Thus, after the satisfactory result of protocol D, with only one sclera, and in view of the need for test reproducibility, a new protocol (E) was tested using whole sclera and greater number (20 scleras). For this, the ideal concentration of DNase/RNase was adjusted for greater process savings and reduction of rotation to improve material exposure to incubation solutions and addition of washes with deionized water for removing residual SDS. This protocol also met the criteria for reduced DNA quantity. From then on, to mimic a large-scale, semi-automated and cost-effective process, new adjustments were made for carrying out decellularization in 100 scleras using a bioreactor (protocol F). This protocol was also proven to be efficient (table 2)

**Table 2. Quantification of genomic DNA (ng/µL) and DNA concentration by milligram of scleras, of the control samples (in natura) and samples decellularized by A-F testing protocols.**

| TESTS | Samples | [ ] ng/µL | [ ]ng/mg sclera | Mean [ ]ng/mg of sclera |
|---|---|---|---|---|
| CONTROL (in natura) | 2/2 | 79.8 | 319.2 | 342.7 |
| | | 100.0 | 366.3 | |
| Protocol A | 1 | 45.5 | 151.6 | - |
| Protocol B | 1 | 41.2 | 147.1 | - |
| Protocol C | 1 | 18.5 | 59.7 | - |
| Protocol D | 1 | 2.4 | 9.6 | - |
| Protocol E | 5/20 | 4.2 | 16.8 | 15.8 |
| | | 3.6 | 12.9 | |
| | | 6.1 | 21.1 | |
| | | 3.8 | 14.5 | |
| | | 4.2 | 13.9 | |
| Protocol F | 15/100 | 6.2 | 23.2 | 23.7 |
| | | 4.9 | 18.6 | |
| | | 8.1 | 25.6 | |
| | | 11.5 | 50.4 | |
| | | 7.1 | 27.0 | |
| | | 6.6 | 28.6 | |
| | | 6.6 | 25.9 | |
| | | 6.8 | 21.9 | |
| | | 5.4 | 16.9 | |
| | | 3.6 | 14.1 | |
| | | 5.1 | 19.3 | |
| | | 6.7 | 28.8 | |
| | | 4.8 | 17.9 | |
| | | 5.6 | 23.1 | |
| | | 3.9 | 14.0 | |

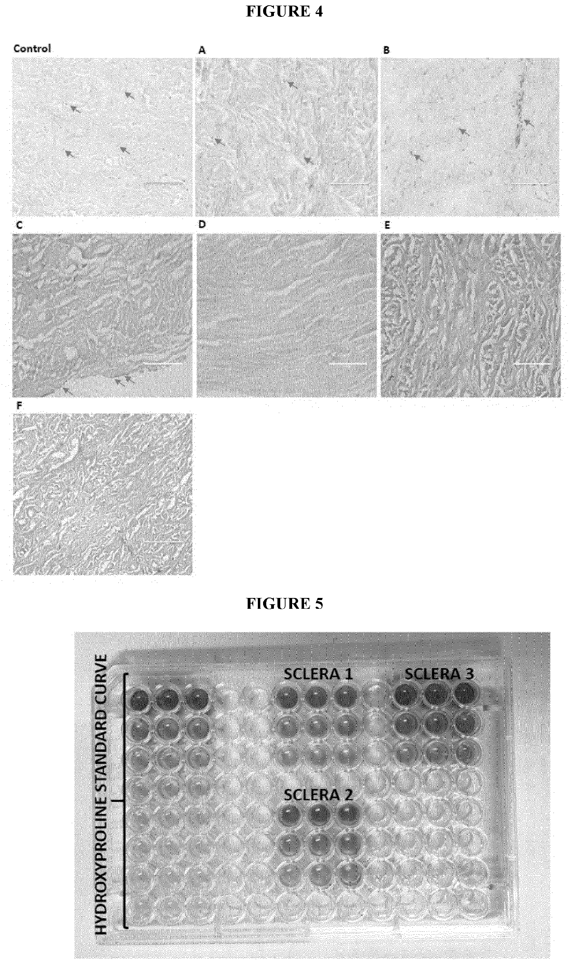

Through histological analysis (HE staining) it was evidenced that the control samples (in natura) have intact nuclei (purple points - arrows show the nuclei). In the evaluation of the images related to protocols A, B and C, nuclei remnants were noted at certain points, demonstrating that these protocols were not efficient. On the other hand, it was not possible to observe, in protocols D, E and F, the presence of nuclei, demonstrating the decellularization efficiency of the material from these tests (figure 4).

According to Gilbert et al. (2006), the effectiveness of decellularization protocols depends not only on competent chemical, enzymatic and physical methods, but also on the origin of the tissue and its composition. The decellularization protocol described by Kasbekar (2018) was standardized for human bulbar connective tissue, while the one proposed herein used animal scleral tissue, which can partly justify the inefficient decellularization through protocol A. Additionally, the process described by Kasbekar (2018) reaches an n sample of 20 eyes, and the present study, through the modifications made, achieved an efficient decellularization of 100 scleras, demonstrating adaptation, time reduction and savings of resources for large-scale production. Also, according to the verification protocols, the reproducibility of the technique can be noted, since it respects the stipulated limits of DNA detection and absence of nuclei in histological analyses.

Thus, the process of standardized final decellularization in this study (protocol F) was more efficient (100 scleras), with better cost-benefit ratio (concentration of DNase and RNase), allowing the reduction of the processing time by number of scleras (semi-automated in a bioreactor), meeting the criteria of evaluation and reproducibility.

### Example 3a - Decontamination protocol of the decellularized biomaterial.

For *in vitro*/*in vivo* tests, the decellularized biomaterial was decontaminated by performing an initial wash in sterile PBS and then soaked in 1x sterile PBS (approximately 30 mL) containing concentrated antibiotic and/or antimycotic solution. Preferably the concentrated solution of antibiotic and/or antimycotic solution comprises at least one compound with antibiotic action and at least one compound with antimycotic action, but it particularly comprises: penicillin, streptomycin and amphotericin B. The samples were stored in this solution for about 36h-60h, preferably about 48 h, at a temperature of about 2°C-8°C, preferably 4°C. After the storage period, fragments of the biomaterial were taken from each sample of the biomaterial and rapid washing was performed in 1x sterile PBS. These fragments were submitted to decontamination testing by their inoculation in Luria Bertani (LB) microorganisms culture medium, without antibiotics and fungicides. These samples were incubated at 37°C under stirring for 72h. For evaluating the presence of some type of contamination by microorganisms, after 72h, the optical densities (OD) (by spectrophotometry, wavelength: 600 nm) of the culture medium where the fragments in question were soaked and of other controls (1x wash PBS, sterile LB medium without any type of inoculum) were measured. Details of the experiment carried out can be found below:

Materials soaked in LB medium for sterility testing:
A) Fragment of decellularized biomaterial
B) Fragment of in natura sclera
C) Fragment of dehydrated sclera
D) 1x PBS from previous wash
E) LB medium only

The results of the decontamination test (OD reading) showed the following results:
A) -0.022
B) -0.018
C) -0.021
D) -0.025
E) 0

The readings did not show growth of microorganisms, and consequently, that the protocol used was able to decontaminate the decellularized biomaterials.

The said method was proven to be effective both as an initial and preliminar decontamination step of the decellularized biomaterial, that can further be subjected to an additional sterilization step (using ethylene oxide or any other method known by one person skilled in the art, for example, gamma radiation) .

### Example 3b - Sterilization protocol of the decellularized biomaterial.

In another embodiment of the present application, the sterilization step was carried out using ethylene oxide. After the decellularization and dehydration steps as described in the present application, the samples of the biomaterial were then cut into specific dimensions for carrying out the tests, and dried at room temperature for about 30 minutes to 1 hour. After drying, they were packed and sealed in surgical grade paper, ensuring identification and sealing to verify ethylene oxide sterilization later.

The samples were then subjected to an ethylene oxide sterilization process and aerated for 1 day. After that, analyzes were carried out to investigate the microbiological growth for 14 days in thioglycolate broth culture medium (30°C to 35°C) and tryptic soy broth - TSB (20°C to 25°C).

There was no macroscopic change in the biomaterial samples compared to before the sterilization process. The sterility of the material was verified after sterilization, in a culture medium after 14 days. For both, the thioglycolate broth and the TSB broth there was no microbiological growth, presenting a negative result and a satisfactory sample regarding its sterility.

Said method was proven to be effective to be used as a decontamination and/or sterilization method of the biomaterial.

### Example 4 - Collagen quantification.

Quantification of hydroxyproline (HP) aims at indirect determination of collagen content of the biomaterial produced. Thus, 5-50 mg of the samples were homogenized in 100-400 µL of deionized water. Subsequently, according to the protocol, an amino acid dilution curve (HP) was performed.

Figure 5 shows on the left side, in triplicate, the standard hydroxyproline curve as control, with decreasing dilutions of this substance in vertical. The darker it is, the higher is the hydroxyproline concentration, which is one of the main components of collagen. On the right side, three samples of individual decellularized biomaterial had the hydroxyproline quantification carried out in 3 decreasing dilutions in vertical.

To each 100-400 µL of the homogenized sample, the corresponding number of pure HCl was added (1:1). Thereafter, the solution was incubated for 3-8 hours at 100-120°C. Then, the sample was vortexed and centrifugated from 2-7 min to remove the precipitate.

The reaction was prepared in 96-well plates, in which 5-50 µL of the samples were added to the wells, as well as 5-50 µL of each solution of the hydroxyproline dilution curve, and the test was performed in triplicate. The plates were dried for removing hydrochloric acid. After drying, 50-500 µL of the chloramine T reagent (citrate-acetate buffer, 50% n-propanol and chloramine T, pH 6.5) were added. The plates were incubated at room temperature from 2-10 minutes.

Finally, 100-500 µL of Ehrlich Reagent (perchloric acid, n-propanol and p-dimethylaminobenzaldehyde - DMAB) were added, which interacts with hydroxyproline in the sample, and consequently causes a colorimetric reaction in which the intensity of staining is proportional to the number of amino acid (HP) in the material. The darker the color, the greater the number of hydroxyproline in the sample. Thus, the sample was incubated at 50-80°C for 60-120 minutes and the absorbance was read at 560 nm. In figure 5 the reaction at the end of the protocol can be seen, right before reading by spectrophotometry.

According to the quantification in fragments of three distinct decellularized biomaterial samples, the mean concentration was 103.76 µg of hydroxyproline per milligram of biomaterial, which corresponds approximately to 769 µg of collagen per milligram of biomaterial. Considering that a freeze dried sclera has an average of 370 mg weight, the collagen content of the decellularized biomaterial produced corresponds to 76.9% its weight.

### Example 5 - Dehydration and rehydration test of the decellularized biomaterial.

This experiment aimed to know and measure the reabsorption capacity of aqueous or alcoholic solutions from the dehydrated biomaterial. For this, fragments of different sizes of the decellularized biomaterial (e.g.: 0.6 x 0.6 cm; 1.0 x 1.0 cm; 1.5 x 1.5 cm) were initially weighed and then dehydrated in ethanol at increasing concentrations (45%, 55%, 65%, 75%, 85%, 95% and 100% volume/volume), incubating them for at least 3 hours in each concentration. Then, after total dehydration, the fragments were again weighed and rehydrated, and incubated for about 20 minutes-40 minutes, with at least one selected substance from the group that consists of: water, alcohol, water or alcohol soluble substances, nutrients, metabolites, growth factors, signaling molecules, regulatory molecules, hormone, medicaments and/or pharmaceutically active compounds.

In the example described herein, the biomaterial was submitted to rehydration by adding fixed volumes of water and incubated for up to 30 minutes at room temperature (with quantification of the volume absorbed every 15 minutes). After rehydration, the fragments were weighed again, and the reabsorbed volume was measured (tables 3 and 4).

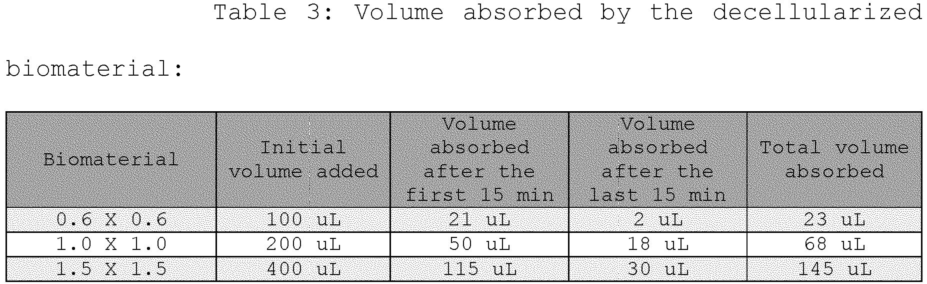

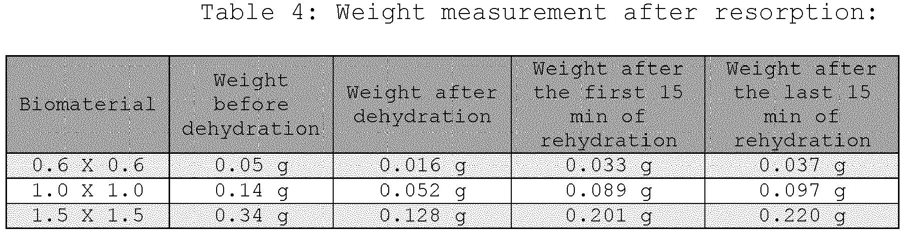

According to the results, dehydrated and rehydrated decellularized biomaterial reabsorbs about 1/4 of the volume applied or approximately 65.4 µL/cm².

In an embodiment of the invention, the dehydration step occurs after the decellularization and decontamination steps of the biomaterial. The decontamination process is carried out with an antibiotic and/or antimycotic solution.

In another embodiment of the invention, the dehydration step can be carried out after the decellularization of the biomaterial, with an initial decontamination step taking place or not, but before subjecting it to the sterilization step. The sterilization process being carried out with ethylene oxide or any other method known by one person skilled in the art, for example, gamma radiation.

The product then obtained by said process comprises a dehydrated, decontaminated and/or sterilized decellularized biomaterial, which can be stored, and can then go through a rehydration and/or association step with at least one active compound only when it is used.

### Example 6 - Analysis of type I collagen, type III collagen and elastin contents.

The objective was identifying, qualitatively, the presence of type I collagen, type III collagen and elastin in the decellularized biomaterial produced. Briefly, three nitrocellulose membranes were sensitized with small fragments of in natura scleras, dehydrated and rehydrated scleras and the decellularized biomaterial produced. Primary antibodies against each protein were used as positive controls of the reactions. The protein-free binding sites present in the nitrocellulose membrane were blocked by the addition of 1-3% PBS-milk buffer, until covering the membranes entirely, under stirring, at 20-40°C, for 1-5h. Then, the membranes were washed 1-7 times with 1x PBS buffer, and each membrane was incubated with a specific primary antibody and diluted in 1x PBS, under stirring at 20-40°C, for 1-5h. After washing 1-7 times with 1x PBS-Tween buffer (PBS-T) at 0.05%-1%, the respective IgG-specific secondary antibodies, marked with peroxidase and diluted in PBS, were incubated, under stirring, at 20-40°C, for 1-5h. Finally, the membranes were washed again 1-7 times with 0.05% PBS-T buffer and the reaction was revealed with DAB (3,3'-diaminobenzidine) solution.

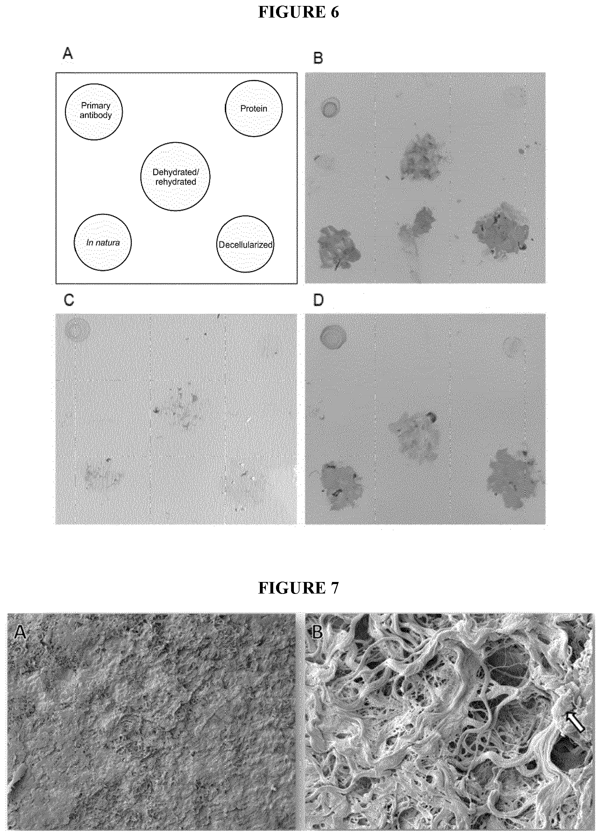

Figure 6 shows the dot blot of the proteins analyzed, showing the presence of each type of protein, with figure 6A showing the experiment layout. Figures 6B, 6C and 6D are representative images of dot blot for type I collagen, type III collagen and elastin, respectively.

According to the dot blot results, in natura sclera, dehydrated and rehydrated sclera and the decellularized biomaterial produced present the three proteins tested: collagen I, collagen III and elastin.

### Example 7- Morphological analysis of biomaterial under scanning electron microscope.

The morphological analysis of the decellularized biomaterial produced aimed to evaluate its three-dimensional structure, the presence and quality of collagen fibers and the verification of the presence/absence of cells in the different materials analyzed.

Samples of in natura sclera, dehydrated sclera, dehydrated and rehydrated sclera and decellularized biomaterial produced were washed with buffer and fixed in a fixing solution suitable for electron microscopy. Then, the fixer was removed, and the samples were submitted to progressive dehydration in solutions with increasing concentrations of ethyl alcohol. After dehydration, the samples were dried and stored. The samples received 3 cycles of gold-palladium cover in the metallizer and were visualized using a Zeiss EVO MA10 microscope. All analyses were performed in triplicate, in several magnifications, using 5-15 kV exposures.

Figures 7A and 7B show scanning electron microscopy of in natura sclera samples, under magnifications of 833 times and 2900 times, respectively. The presence of cells can be clearly visualized as indicated by the arrow in figure 7B.

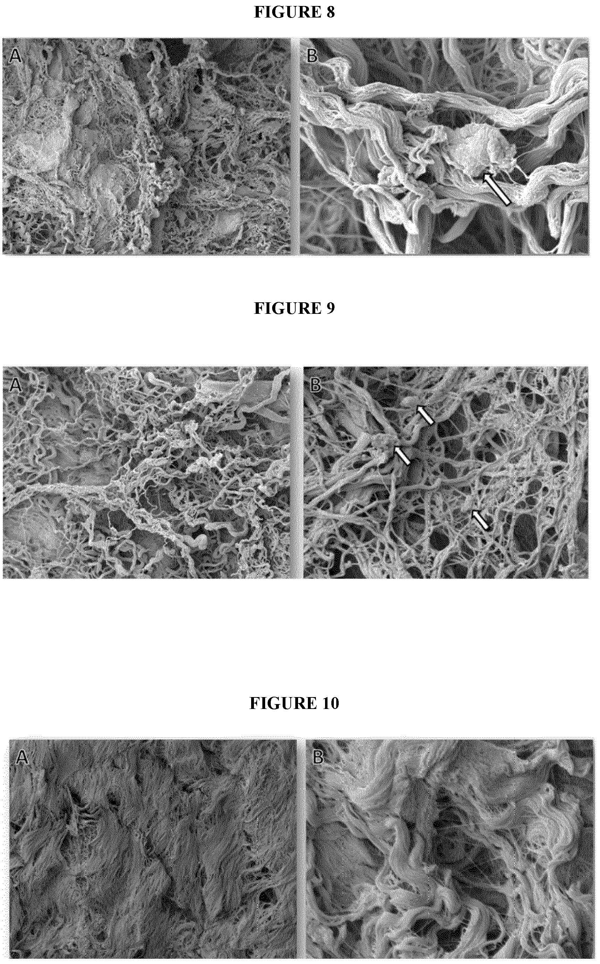

Figures 8A and 8B show scanning electron microscopy of dehydrated sclera samples, under magnifications of 833 times and 7100 times, respectively. The presence of cells can be clearly visualized as indicated by the arrow in figure 7B.

Figures 9A and 9B show scanning electron microscopy of dehydrated and rehydrated sclera samples, under magnifications of 1250 times and 2640 times, respectively. The presence of cells can be clearly visualized as indicated by the arrows in figure 7B.

Figures 10A and 10B show scanning electron microscopy of samples of the decellularized biomaterial produced, under magnifications of 923 times and 5520 times, respectively. The presence of cells has not been identified, and the collagen and elastin fibers remained without apparent or relevant alteration.

Electron microscopy analyses showed that the decellularization method used, led to cell removal from the biomaterial, while in the other groups where the biological material did not go through the decellularization process, the presence of cells was clearly observed. In addition, the fibers were kept healthy in the decellularized biomaterial, showing that the biomaterial comprises a decellularized fibrous matrix that maintained the characteristics of a biological matrix without the cell's removal presenting relevant alterations in the formation and quality of the fibrous matrix, when compared to in natura, dehydrated or dehydrated/rehydrated sclera.

### Example 8 - Biomechanical assay (elasticity and maximum strain modules).

A biomechanical assay was performed to assess the resistance of scleras and the produced biomaterials under different conditions, in order to evaluate the physical properties of this biomaterial and enabling the indication of possible applications in the context of regenerative medicine. For this assay, approximately 12 fragments of sclera samples (from 12 different individuals) were used under the following conditions: in natura sclera, decellularized biomaterial produced, dehydrated decellularized biomaterial (in increasing concentrations of ethanol) and dehydrated and rehydrated decellularized biomaterial (dehydrated in increasing concentrations of ethanol and rehydrated in water for 30-50 min). All fragments were obtained from the central region from each sclera in natura, with approximate measurements of 5 x 20 x 1 mm (width x length x thickness). The specimens (fragments) were submitted to a tensile assay using a WDW 38/56 - 1000E Universal Test Machine from TIME Group Inc. To achieve the forces exerted, a load cell with a maximum capacity of 50 kgf was used and the deformations were registered by the machine's internal displacement sensors. The specimen was fixed in two claws apart from 5 mm. The force application speed was 2 mm/min.

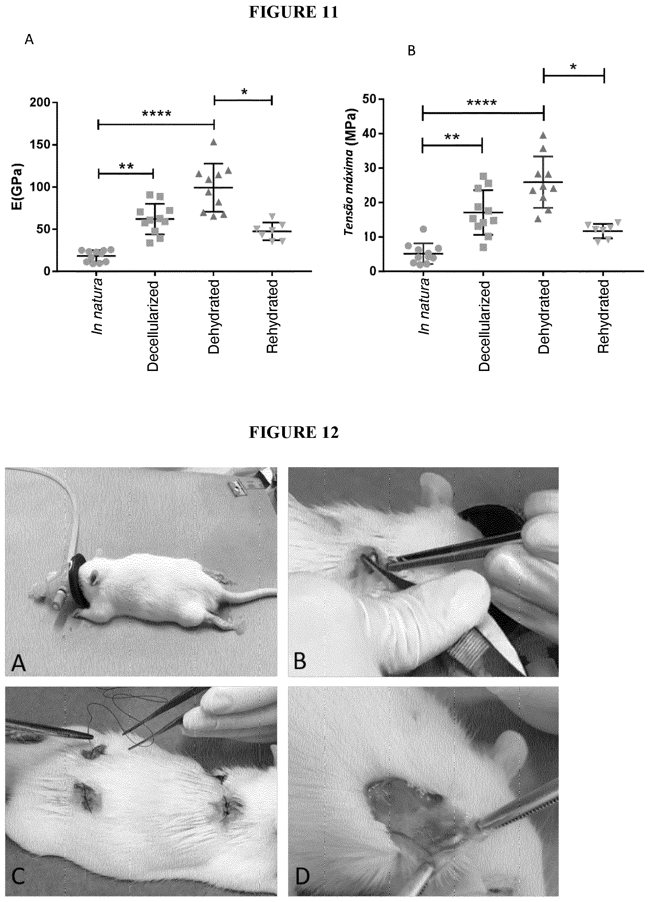

Figure 11A shows a scatter dot plot of the Young elasticity module (GPa) for in natura sclera samples (In natura), samples of decellularized biomaterial (Decellularized), samples of decellularized and dehydrated biomaterial (Dehydrated) and samples of decellularized, dehydrated and rehydrated biomaterial (Rehydrated). The Young elasticity module used in this study describes the ratio between strain and deformation in the direction of the applied load, being the maximum strain that a material supports without undergoing permanent deformation. Thus, it was possible to note through the elasticity parameter (E) that samples of dehydrated decellularized biomaterial and decellularized biomaterial presented significantly greater stiffness when compared to the in natura sclera sample. In addition, the sample of dehydrated decellularized biomaterial also showed significantly higher stiffness compared to the dehydrated and rehydrated decellularized biomaterial sample.

Figure 11B shows a scatter dot plot of the maximum strain (GPa) for in natura sclera samples (In natura), samples of decellularized biomaterial (Decellularized), samples of decellularized and dehydrated biomaterial (Dehydrated) and samples of decellularized, dehydrated and rehydrated biomaterial (Rehydrated). As expected, the same behavior was observed for the maximum strain parameter (MPa), which describes the maximum strain that the material still supports in the elastic deformation regime. Thus, due to their greater stiffness, samples of dehydrated decellularized biomaterial and of decellularized biomaterial withstood significantly higher strains when compared to in natura sclera. The dehydrated decellularized biomaterial, by its turn, still withstood a significantly higher strain compared to dehydrated and rehydrated decellularized biomaterial.

It is important to highlight that samples of the biomaterial produced, regardless of the type of treatment analyzed (Decellularized, Dehydrated or Rehydrated), presented higher resistance (higher Mpa) in relation to in natura samples. Despite supporting a greater force applied in their area, they also presented a small relative deformation, which was close to the value presented by the in natura sample. Such characteristics confirm the potential of varied applications for the biomaterial produced, such as: in tissue repair and/or regeneration processes, preferably in bone tissue, cartilage tissue, joints, tendons, skin, for prosthetic purposes in implantology and/or oral and bucomaxillofacial traumatology, for surgical filling and/or anatomical correction procedures, or in the production of prostheses.

### Example 9 - Subcutaneous implantation of biomaterial produced to analyze local inflammation.

To assess the inflammatory response due to the presence of biomaterial in experimental animals, we implanted the biomaterial subcutaneously in the dorsal region of rats.

The animals were anesthetized with isofluorane, trichotomy and antisepsis were performed in the region and, soon after, longitudinal incisions with a scalpel were performed. The subcutaneous region was divulsed using a blunt-tip scissors and then a fragment of the decellularized biomaterial of 0.5 cm x 0.5 cm x 0.2 cm was introduced in one of the incisions and, in another incision, only the divulsion of the tissues was made without introducing biomaterial and used as a control. The incisions were sutured, and the animals were kept under observation until the end of anesthesia. Animal euthanasia was performed after 5 and 15 days (acute and chronic inflammation, respectively) of the material implantation. The implantation region was removed, fixed in 10% formaldehyde for histological analysis and stained with HE (images representing this procedure are shown in figure 12). Histological examination aimed to evaluate the intensity of inflammatory reaction of surrounding tissue, presence of vessels, fibroblasts, multinucleated giant cells and tissue integration.

Figures 12 A-D illustrate the procedure for implanting the biomaterial produced in subcutaneous tissue in rats. Figure 12A shows the animals under anesthesia with isofluorane mask. Figure 12B shows the implant of the biomaterial. Figure 12C shows the suture of the incisions. Figure 12D shows the removal of the implantation region after 5 or 15 days of surgery.

To carry out the analysis of the images, six fields of histological cuttings were selected and classified with scores according to the presence and intensity of acute and/or chronic inflammation, fibroblasts, vessels and multinucleated giant cells, according to the table below.

**Table 5: Analysis parameters score**

| Score | Assessment |
|---|---|
| 0 | Absence |
| 1 | Rare and weak |
| 2 | Occasional and attenuated |
| 3 | Frequent and localized |
| 4 | Frequent and diffuse |

The analyses were performed by 4 independent evaluators in all selected fields. The mean of the scores of each animal was calculated, assigned by each evaluator and a comparison between the results of 5 and 15 days was performed.

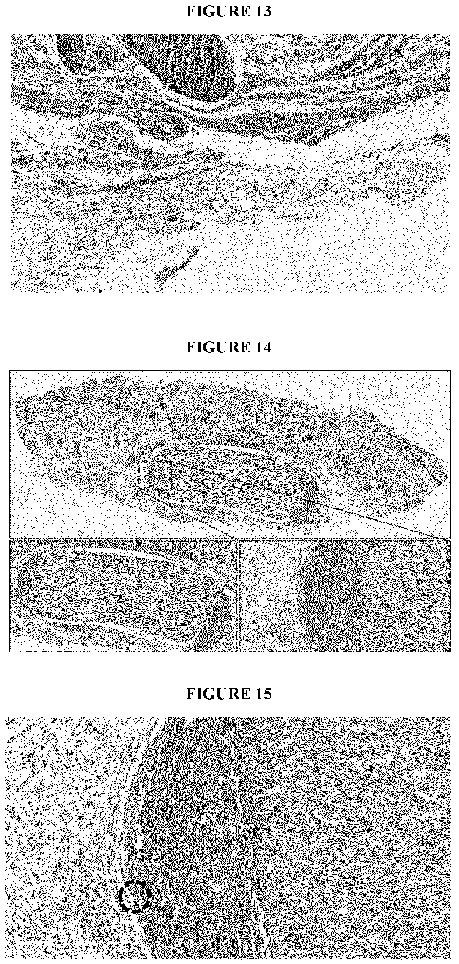

Figure 13 shows a representative image of the histological cutting from the control group after 5 days. On the other hand, figure 14 shows representative images of the histological cutting of the subcutaneous biomaterial implant after 5 days of implantation. The insert shows a specific field, showing the contact area of the inflammatory infiltrate with biomaterial.

Figure 15 shows a representative image of the histological field of the implant of subcutaneous biomaterial after 5 days of implantation. Arrows indicate fibroblasts infiltrated in the biomaterial (material evidenced on the right side) and the dotted circle is an example of blood vessel.

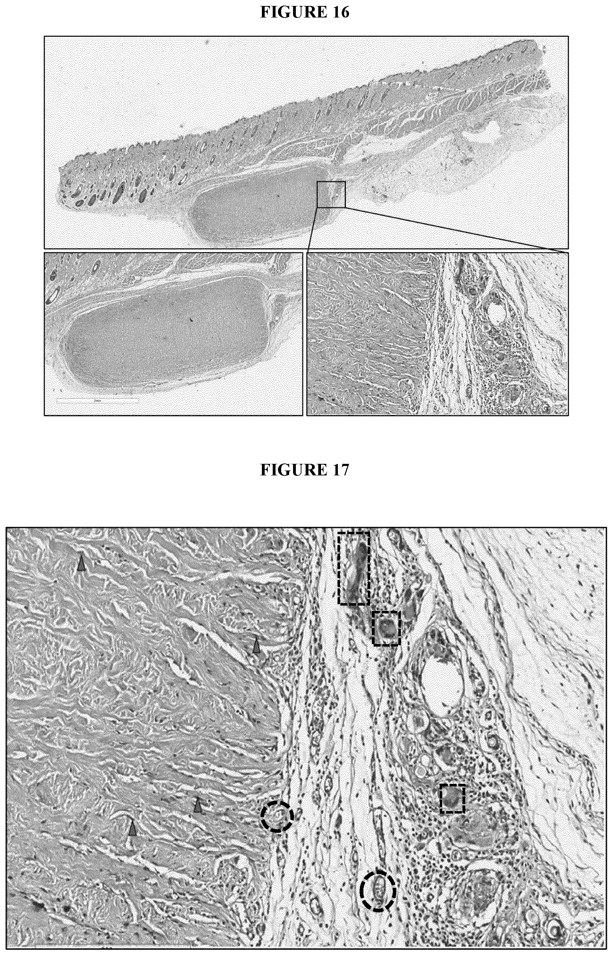

Figure 16 shows representative images of the histological section of the subcutaneous biomaterial implant after 15 days of implantation. The insert shows a specific field, showing the contact area of the inflammatory infiltrate with the implanted biomaterial.

Figure 17 shows a representative image of the histological field of the subcutaneous implant of biomaterial after 15 days of implantation. The arrows indicate fibroblasts infiltrated in the biomaterial (material evidenced on the left side). The dotted circles indicate blood vessels. The dotted rectangles indicate multinucleated giant cells.

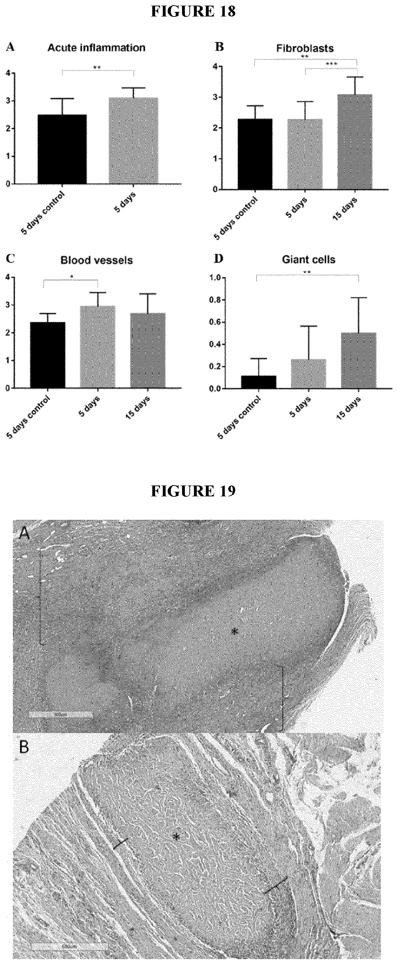

Using the visual score predetermined by the research group, histological analysis suggests interaction of the biomaterial implanted with adjacent tissue, without showing signs of severe rejection. Figures 18 A-D show a statistical analysis of the evaluated parameters (***: p = 0.0006; **: p = 0.0015; *: p = 0.038).

There is a significant difference in the comparison of the intensity of acute inflammation, being higher in the group of 5 days after implantation, when compared to the respective control (figure 18A). Regarding the presence of fibroblasts, there was a significant increase in the number of these cells in the group of 15 days after implantation, both compared to the group of 5 days after implantation and to the control group of 5 days (figure 18B). Vascularization, by its turn, showed a significant increase in the 5-day post-implant group when compared to its respective control group (figure 18C), while the presence of giant cells showed a significant increase in the 15-day post-implant group compared to the 5-day control group (figure 18D). Although there is no statistical difference in the analysis related to the presence of vessels, it was visually possible to note a neovascularization inside the biomaterial, after 15 days of implantation, which suggests integration by the body. Additionally, the presence of a greater number of fibroblasts migrating into biomaterial after 15 days of implantation suggests that there is remodeling and/or tissue degradation in the peripheral region to the biomaterial.

According to the results related to subcutaneous biomaterial implantation, there was no induction of exacerbated inflammatory response, and the inflammatory process detected is important for gradual tissue remodeling, since the presence of fibroblasts increases when compared to groups of 15 and 5 days, enabling deeper migration and possibly characterizing the integration of biomaterial implantation into local tissue. Signs of severe rejection were not detected during the experiment execution time, in accordance with histological and macroscopic analyses when removing the material after experimental time, suggesting that the biomaterial presents promising characteristics for use in tissue engineering and regenerative medicine.

### Example 10- Implantation of the biomaterial in a tendon injury.

To demonstrate the potential use of decellularized biomaterial in tissue repair and/or regeneration, *in vivo* implantation experiments of the said biomaterial were performed.

The experiment sought to analyze the effect of implanting the dehydrated decellularized biomaterial produced in ram tendon injury.

For the study, male rams aged between 2-4 years were used. All animals were subjected to general anesthesia and all the aseptic, ethical and technical care required by a surgical procedure were used. After removing a fragment of the tendon, the implant made of the biomaterial produced by the method described in the present application was placed and fixed to the bone with a titanium screw and suture at the end of the tendon.

The postoperative period was monitored and the animals received antibiotics, anti-inflammatories and analgesics. During follow-up, videos were made to assess claudication (gait impairment assessment), 48 hours after surgery and periodically until euthanasia.

The animals were randomly divided into two groups with five animals each, to be euthanized in the first experimental time of 49 days (D49) and second experimental time of 114 days (D114) postoperatively. On the day of euthanasia, the animals were clinically assessed, presenting normal mucosa, vital signs and behavior.

After the incision, in the same region as the previous surgery, the surgical specimen was collected and stored in a solution containing 10% formaldehyde and sent for histological slides stained with hematoxylin and eosin.

Claudication assessment and histological analysis were performed at both experimental times (D49 and D114) . Based on the evaluations of all animals, only one animal in the 49-day group showed worsening in claudication, the others presented favorable and positive performance in walking. In addition, histological analysis of tissue and implant was performed at both experimental times, that showed an interaction of host cells with the peripheral region of the implant.

### CLAUDICATION ASSESSMENT OF ANIMALS.

To assess claudication, the following parameters were considered: absent, when the animal trots without claudication; light, when the animal walks with slight imbalance; moderate, when claudication is interspersed with walking; severe, when the animal cannot steady its paw while walking.

Considering the animals from both experimental times (D49 and D114), the first claudication assessment was performed 48 hours after the surgical procedure. In this analysis, only one animal did not present severe claudication. After 15 days of the surgical procedure, all animals presented good gait evolution. In the assessment after 30 days, two animals showed worsening in claudication, presenting stumbling, one from D49 and the other from D114. The others had mild claudication or maintained the previous pattern. After 45 days, all animals presented relative improvement compared to the last analysis, except for one animal in the D114 group that again had difficulty steadying its paws.

On the day of euthanasia of the first group (D49), only one animal evolved with worsening claudication. The other animals could walk without claudication or trot without severe imbalance. The assessments continued with the animals from the second experimental period and until the day of euthanasia (D114), they maintained the parameter of mild/absent claudication when trotting.

Based on the assessments performed at the two experimental times, when comparing the integral evolution of the analyzes (day of surgery to the day of euthanasia), worsening of gait was observed in only one animal in the D49 group. All other animals had a good outcome with recovered gait function.

### HISTOLOGICAL ASSESSMENT

After the qualitative histological assessment of the D49 group, it was possible to observe the presence of an intense and diffuse inflammatory process around the implant. There was apparently an interaction between the implant of the decellularized biomaterial and the host cells, which migrated between the collagen fibers at the periphery of the implanted tissue (Fig. 19A).

Figure 19 (A) shows the diffuse inflammatory infiltrate and implanted decellularized biomaterial in the D49 experimental group and Figure 19 (B) shows the less diffuse and more defined inflammatory infiltrate (indicated by { }) around the implanted decellularized biomaterial (indicated by *) in the second experimental group D114.

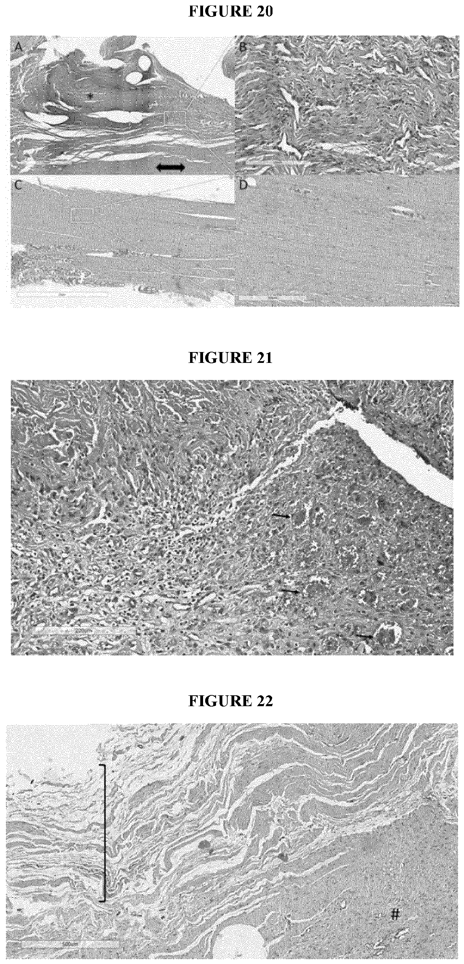

The region between the injured tendon and the implant presented disorganized, hypercellularized fibers, different from what is observed in a healthy native tendon (Fig. 20).

Figure 20 (A) shows the region of the implanted biomaterial (indicated by *); injured native tendon (indicated by ↔) and region intermediate to the native tendon and the implant (highlighted). Figure 20 (B) shows the tissue adjacent to the injured native tendon, with disorganized and hypercellularized fibers. Figure (C) shows the healthy native tendon that was removed for replacement by the implant. Figure (D) shows a region of healthy native tendon at higher magnification.

It was possible to notice in most of the analyzes the presence of neovascularization, and also, the increase in the number of cells inside the blood vessels, in regions close to the implanted tissue (Fig. 21). We also visualized some regions of neo-formed tissue, with characteristics similar to loose connective tissue, close to the tendon defect.

Figure 21 shows the presence of blood vessels and hyperemia in the region of interaction of the native tissue with the biomaterial implant in the first experimental time D49 (indicated by the arrows).

In some samples, in the region external to the implant and to the injured tendon, it was also possible to macroscopically observe a neo-formed tissue, covering all the extension of the lesion, and this tissue microscopically presented characteristics of a loose connective tissue (Fig. 22) .

Figure 22 shows a microscopic image of the loose connective tissue (indicated by ]) overlying the injured tendon region (indicated by #).

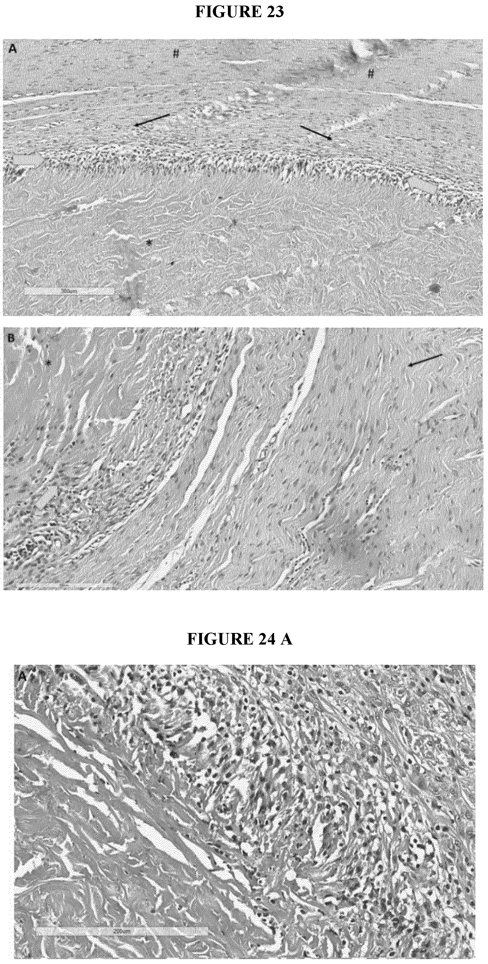

In the second experimental time D144, it was possible to observe the presence of the inflammatory infiltrate, but less diffusely and more defined around the implant, with a smaller number of cells (Fig. 19B). In addition, in some slides, the presence of some regions of neo-formed and/or remodeled tissue together with the implant was found, showing an apparent deposition of extracellular matrix and an increase in the number of fibers, more compact and parallel in the region of the defect in continuity both with the implant and with the native tissue, which suggests a gradual tissue remodeling with a discreet intention of organizing its fibers in the same direction as the fibers of the native tendon (Fig. 23 A and B).

Figures 23 (A) and (B) show the decellularized biomaterial implant in interaction with the native tendon in the second experimental time. The implant (indicated by *) is observed to be infiltrated by host cells; with a predominance of those with fibroblast characteristics (indicated by thick arrows). The fibers in the implant-tendon interaction region (indicated by the black arrows) tend to organize themselves with the increase in compaction and the parallel arrangement between them; becoming, in this region of the implant, with histological aspect of tissue organization similar to that of the native tendon (indicated by #).

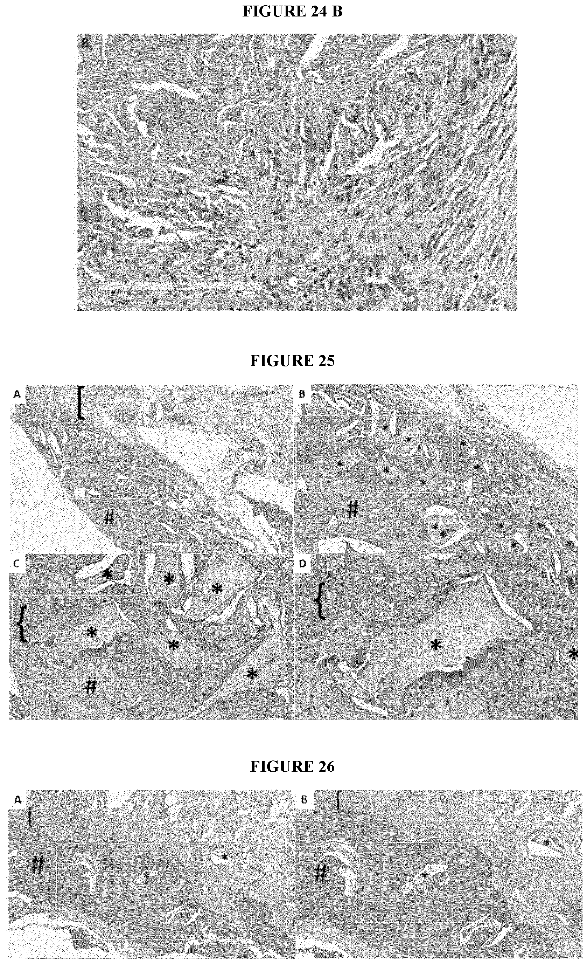

In general, in this second experimental time it was possible to observe the predominance of fibroblast-like cells infiltrating the implant tissue, different from the cells that were observed in the first experimental time (Fig. 24 A and B). It suggested the occurrence of intense acute inflammation in the initial time, with reduction of inflammation in the second time, showing a decrease and alteration of cell types and a tendency to repair the lesion.

Figures 24 (A) and (B) show the profile of predominant cells that infiltrated the implant: first experimental time D49 (A) and second experimental time D114 (B) .

Based on the histological findings and comparative evaluation between the two experimental times, it can be inferred that the decellularized biomaterial implant produced by the method described in the present application acted efficiently to allow cellular infiltration and also provided a favorable environment so that these cells used this scaffold in an attempt to induce repair and/or remodeling of the lesion, although the experimental time was not sufficient to visualize the complete integration of the implant or complete repair of the lesion. In addition, the clinical, macroscopic and histological aspects showed no signs of infection and/or rejection of the biomaterial, suggesting a good interaction of this implant during the assessed experimental times.

The decellularized biomaterial implant showed the potential for tissue remodeling, allowing recellularization mainly in its periphery and allowed the vascularization of the region and favored the deposition of extracellular matrix, which are essential factors for the remodeling of the injured tissue

### Example 11- Implantation of the biomaterial in the bone.

To verify the potential of using the biomaterial as a graft in a tissue repair and regeneration process, and also as a carrier of active substances, an *in vivo* bone implant experiment was carried out, more specifically, an implant on the buccal surface of the maxillary alveolar bone of dogs.

Male and female dogs, aged between 2-8 years, were used. All animals were submitted to general anesthesia and all the aseptic, ethical and technical care required by a surgical procedure were used.

The assay consisted of three experimental groups. In the first group called CONTROL, the standard commercialized material for bone regeneration was used, 0.25 g Geistlich Bio-Oss^{®}; in the second group, the decellularized dehydrated biomaterial, called BIOMATERIAL group, was used; and in the third, the decellularized dehydrated sclera, carrying 2.5% simvastatin, called BIOMATERIAL WITH ACTIVE COMPOUND group.

Longitudinal incisions were made with a No. 15 scalpel blade on the attached gingiva and later the periosteum was incised and detached along the bone area to be exposed, thus allowing direct access to the alveolar bone. Grooves were made on the recipient bone surface with an average size of 0.5 x 0.75 cm with a conical carbide surgical bur #701, always with external irrigation with concomitant and uninterrupted saline solution to prevent overheating of the bone portion. After positioning the biomaterial over the grooves, the periosteum and gingiva were repositioned for surgical synthesis, using isolated stitches, with monofilament nylon thread (4-0), mounted on a 1.5 cm long semicircular atraumatic needle. After the surgical procedure, the animals received doses of antibiotic, anti-inflammatory and analgesic for three consecutive days.

Fragments from the surgical site were removed after 49 (D49) and 130 (D130) days. The samples were fixed in a solution containing 10% formaldehyde, dehydrated in increasing concentrations of ethanol, diaphanized in xylene, infiltrated, embedded in paraffin and stained with hematoxylin and eosin for subsequent histological analysis under a light microscope.

### HISTOLOGICAL EVALUATION

According to the qualitative histological evaluation on the representative slides of the D49 CONTROL group, it was observed the formation of bone blastemas in the inserted gingiva membrane, surrounding the biomaterial fragments.. The blastema area is compatible with the beginning of the bone tissue formation process, with the presence of typical cells, such as osteocytes, osteoblasts and osteoclasts. It is possible to verify the prematurity of the process, from the analysis of the disorganized distribution of collagen fibers and the presence of osteocytes and osteoblasts and blood vessels inserted in the matrix, findings common to immature bone tissue (Figure 25).

Figures 25 show the oral mucosa membrane with the presence of granules from the implanted commercial bone graft (indicated by *). In (A) it is possible to observe the dense connective tissue where the graft is implanted (indicated by #) and loose connective tissue at the top (initiated by a square bracket). In (B), the commercial graft granule is highlighted (indicated by *) with the formation of the adjacent bone blastema, this process being enlarged and detailed in (C) and (D), in the control group D49.

There was also a slight increase in the number of blood vessels close to the granules of the commercial bone graft and mild hyperemia, a condition that could favor tissue nutrition and, consequently, contribute to the development of the tissue in question. Thus, it is inferred that the presence of granules induced bone formation, with irregular extracellular matrix, typical of immature bone.

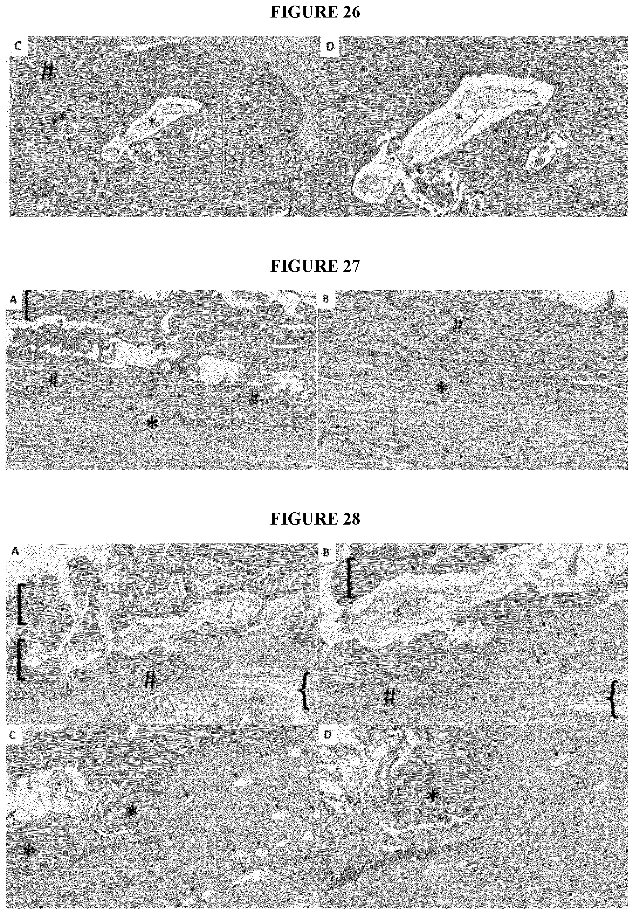

At the experimental time D130 of the CONTROL group, a similar response to the first experimental time was observed, with primary bone containing a large number of osteocytes within the neo-formed matrix and more organized regions, with an arrangement resembling the formation of Haversian Systems close to the granules of the commercial graft. (Figure 26)

Figures 26 show the D130 control group. In (A) there is the presence of commercial bone graft granules (indicated by *) in the membrane, external to the periosteum of the alveolar bone (indicated by [), in addition to the presence of the highlighted granule included in the newly formed bone (indicated by #). The area highlighted in (B) was enlarged in (C) for analysis, where growth lines are visualized (indicated by arrows) close to the biomaterial region and the formation of Havers Systems (indicated by **). At higher magnification, deposition of more organized and oriented collagen fibers can be seen around the particles, as well as osteogenic cells around the biomaterial (D).

In the BIOMATERIAL D49 group, it was possible to observe the intimate contact of the decellularized biomaterial with the alveolar bone of the animal, with a high number of cells interspersing the sclera region and angiogenesis within the biomaterial, characteristics that were remarkable at this experimental time. Areas with significant inflammatory infiltrate were not visualized (Figure 27).

Figures 27 show the BIOMATERIAL group D49. In (A), the attached gingiva membrane region and bone tissue of the experimental animal (indicated by [), implantation of decellularized biomaterial (indicated by *) adjacent to the alveolar bone of the animal (indicated by [). In (B), by magnification of (A), details of the implant and bone tissue in formation (indicated by #) can be observed, where it is possible to verify the interaction of the biomaterial with the bone and probable osteoprogenitor cells invading the biomaterial and the presence of cells in the decellularized biomaterial as well as blood vessels (indicated by arrows).

In the BIOMATERIAL D130 group, the presence of numerous blood vessels is evident, proposing moderate angiogenesis inside the decellularized biomaterial implant and intimate contact of the referred implant with a region that suggests discrete bone formation. Bone formation showed a disorganized or slightly organized pattern, not differing from the initial time, D49. In the biomaterial, interspersed cells were also observed inside, and in the region adjacent to the bone of the experimental animal (Figure 28)

Figures 28 show the BIOMATERIAL group D130. In (A) areas of close integration are observed between the biomaterial implant and the animal's bone (indicated by [) and implant and loose connective tissue (indicated by {). The decellularized biomaterial implant (indicated by #) now has possible osteoprogenitor cells inside and the presence of a small cellular infiltrate interspersing its fibers. In (B), the presence of possible adipocytes is highlighted, with probable origin from the bone marrow (indicated by arrows). In (C) and (D) we can see the deposition of the newly formed bone (indicated by *) on the implanted biomaterial.

Knowing that angiogenesis and cell migration are important in the bone tissue repair process and that osteoconductive biomaterials are used to direct cells more quickly and appropriately in bone repair, an osteoconductive role of decellularized biomaterials is suggested as produced by the method described by the present patent application.

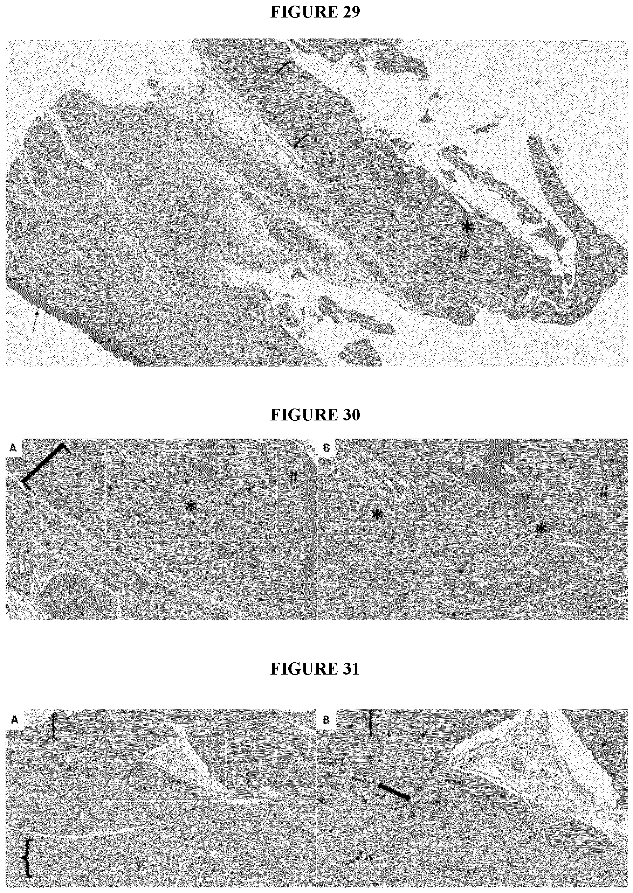

In the BIOMATERIAL WITH ACTIVE COMPOUND group, where the ability of the biomaterial to be used in combination with active substances was assessed, also working as a carrier, in this case using simvastatin, the histological assessment showed the presence of greater bone volume compared to the other groups (Figure 29), where it is possible to observe the alveolar bone and adjacent areas of new bone formation.

Figure 29 shows the group BIOMATERIAL WITH ACTIVE COMPOUND D49. Where the image is a photomicrograph demonstrating the presence of the gingival mucosal epithelium (indicated by an arrow), membrane (indicated by [) of the mucosa and the region where the biomaterial with simvastatin was positioned (indicated by { ) adjacent to the alveolar bone of the animal (indicated by *). The region of bone neoformation (#) is highlighted in the rectangle.

The area of bone formation in the BIOMATERIAL WITH ACTIVE COMPOUND D49 group showed integrated bone trabeculae, characteristic of the bone maturation process. In addition, it is observed in an immature bone region, with a large number of osteocytes and the presence of small medullary cavities containing blood vessels (Figure 30).

Figures 30 show the group BIOMATERIAL WITH ACTIVE COMPOUND D49. Region of interaction between the biomaterial with simvastatin and alveolar bone (A) and connective tissue (initiated by [). There is a greater volume of newly formed bone (indicated by *) adjacent and integrated with alveolar bone (indicated by #), cellular infiltrate and blood vessels within the newly formed bone trabeculae (indicated by *). Newly formed bone has disorganized collagen fibers as shown in image (B) and growth lines (indicated by arrows in A and B) that distinguish it from alveolar bone.

In the second experimental time of the BIOMATERIAL WITH ACTIVE COMPOUND group (D130) a similar behavior was observed in relation to the D49 time, and the presence of a greater neo-produced bone volume, adjacent and delimited by the alveolar bone growth line (Figure 31). In addition, the presence of mast cells in the region close to the biomaterial is also observed, demonstrably related to the tissue remodeling process, as well as the greater presence of blood vessels and cells interacting with the biomaterial implant.

Figures 31 show the group BIOMATERIAL WITH ACTIVE COMPOUND D130. In (A) and (B) we can see the presence of mast cells (indicated by ↔) and other cell types inside the biomaterial with simvastatin. Lines of growth (indicated by arrows) separate mature alveolar bone (indicated by [) from newly formed (indicated by *). Connective tissue from the animal itself (indicated by {).

The decellularized biomaterial produced by the method described in the present patent application had an osteoconductive and osteoinductive role, in addition to favoring cell migration. In addition, it demonstrated the possibility of being used in combination with active compounds, for example by delivering compounds directly to the implant site. Simvastatin is a drug used in the treatment of hyperlipidemia and has pleiotropic osteogenic and antiresorptive effects already studied *in vitro* (showing changes in cellular dynamics resulting from the effects of simvastatin on bone tissue, which are associated with increased expression of bone morphogenetic protein-2 (BMP-2), stimulating the proliferation and differentiation of osteoblasts). Thus, the decellularized biomaterial implant favored bone formation and when associated with simvastatin it presented better performance compared to the other experimental groups.

### Example 12- Implantation of the biomaterial in cartilage lesion.

In order to verify the potential of the use of the biomaterial as a graft, biological scaffold, in a tissue repair and regeneration process, an implant of the decellularized biomaterial produced by the method described by the present patent application in injury caused in cartilaginous tissue *in vivo,* more specifically an implantation of the biomaterial in a lesion caused in the knees of sheep.

Male rams aged approximately 18-month-old and body weight between 48-56kg were used. The animals were submitted to the anesthetic protocol, all the asepsis, ethical and technical care required for a surgical procedure, as well as the medial femorotibial patellar arthrotomy protocol, with the patella retracted laterally and the femurotibial joint flexed. A bone bur was used for joint wear of 6 mm in diameter and approximately 3 mm in depth, until the exposure of the subchondral bone. A 7mm diameter membrane fragment was adapted and inserted into the lesion site. After raffia and post-anesthetic recovery, the animals were sent to the stalls, followed up daily with clinical examinations and immobilized with bandages for 5 days.

After 30 days of the surgical procedure, the animals were euthanized by pre-anesthetic medication and the surgical site was removed. The surgical specimen was fixed in 4% formalin and demineralized with formic acid solution. The procedures for performing the histological slides were followed, stained with Hematoxylin and Eosin.

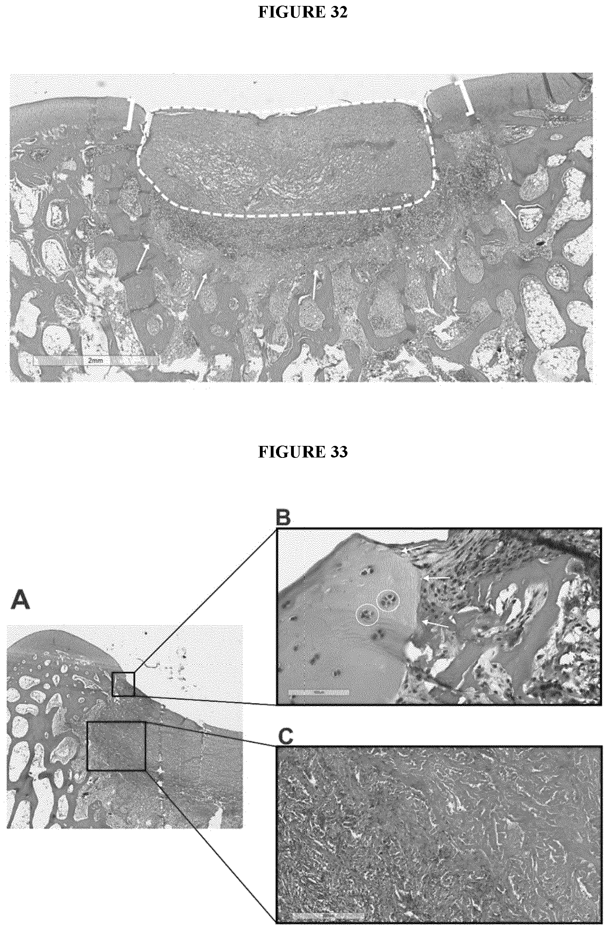

In the histological analysis, it was possible to observe (Figure 32) a good interaction of the implanted biomaterial, but particularly of the collagen fibers of the biomaterial implant with the native bone tissue.

Figure 32 shows a histological section of the group that suffered cartilage injury and received the implant, at an experimental time of 30 days post-surgery. The dashed line indicates the approximate delimitation of the implant at the lesion site. The square bracket (]) indicates the region of native articular cartilage and the arrows illustrate the interaction with bone tissue (subchondral region).

At the ends of the biomaterial implant, intense infiltration of fibroblasts from the animal was observed, accompanied by deposition of new extracellular matrix, which led to the absence of exact delimitation of the edges, demonstrating important integration of the biomaterial implant to adjacent tissues (Figure 33C).

Figures 33 show a histological cutting of the group that suffered the cartilage lesion and received the implant, at an experimental time of 30 days post-surgery. Figure (A) is a representative image of the histological cutting with the demarcated regions that have been enlarged for a better analysis in (B) and (C). In figure (B), the arrows indicate the region of integration between the articular cartilage and the neo-formed connective tissue, indicating important integration with the implant. Circles indicate chondrocyte mitosis figures. Figure (C) shows the region of the biomaterial implant with fibroblast infiltration, deposition of new extracellular matrix, which leads to the loss of border delimitation, due to good integration between the tissues (lower left).

Adjacent to the biomaterial implant, inflammatory cells were also found, with a predominance of mononuclear cells, such as lymphocytes, macrophages and plasma cells, which corresponds to a stage of the repair process. There were no signs of rejection of the biomaterial, such as the presence of multinucleated giant cells (Langerhans giant cells) or encapsulation process, in any of the animals analyzed.

On the upper surface of the implant, a newly formed connective tissue was observed, with important integration with the cartilaginous tissue. In this region, the presence of chondrocyte mitosis figures was observed at the edge of the lesion, demonstrating cartilaginous proliferation, which composes the tissue repair potential of the biomaterial implant produced by the method described by the present patent application (Figure 33B).

### Example 13- Implantation of the biomaterial for filling and anatomical correction.

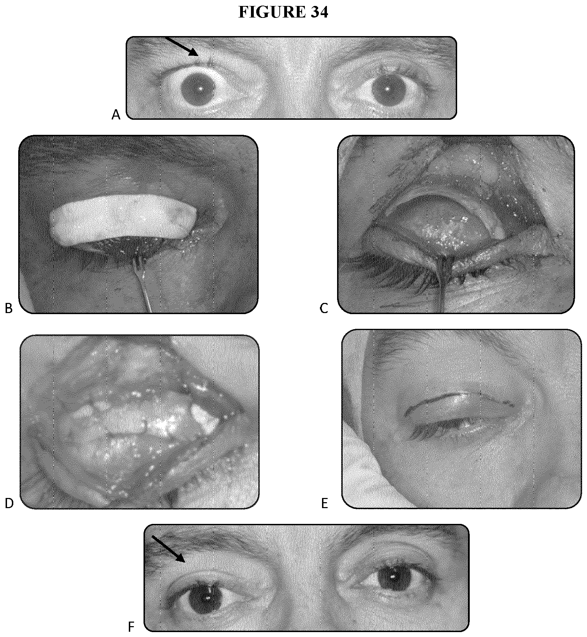

To verify the potential use of the biomaterial in a filling and anatomical correction process, an implant of the biomaterial produced in a human being who had eyelid retention in one eye was performed (indicated by an arrow in Figure 34 A).

The biomaterial implant was then prepared in the proper size to be surgically implanted in the desired eyelid (Figure 34 B-D).

Figure 34 (E) shows the result of the surgery right after the procedure. Figure 34 (F) shows the result of the filling surgery and anatomical correction using the decellularized biomaterial of the present patent application, after approximately 10 months of the aforementioned surgical procedure. The patient did not reject the implanted biomaterial, and the filling and anatomical correction was a success, aesthetically recovering the symmetry of the patient's eyes.

Thus, this application describes a method of producing decellularized biomaterial, comprising the steps of:
- collecting, processing and cleaning biological tissue, preferably when the biological tissue is derived from eye's connective tissue, preferably scleras of porcine, bovine, rabbit or human origin;
- subjecting the obtained decellularized biomaterial to a decellularization process, comprising:
   a - washing the biological tissue 1-5 times using isotonic buffer between 1-20 minutes, at a temperature of 0-25°C, under stirring between 80-200 rpm;
   b - incubating the biological tissue using hypotonic buffer for 1-18 hours at a temperature between 4-37°C under stirring between 80-200 rpm;
   c - treating the biological tissue with detergent buffer, for 5-24 hours, at a temperature between 4-37°C under stirring between 80 and 200 rpm;
   d - washing the biological tissue with deionized water for 30 minutes-4 hours, at a temperature between 4-37°C under stirring between 80-200 rpm;
   e- washing the biological tissue 1-5 times with isotonic buffer, for 5-15 minutes, at a temperature between 4-37°C under stirring between 80-200 rpm;
   f - incubating the biological tissue with nuclease buffer, between 30 minutes-3 hours, at a temperature of 30-40°C, under stirring between 80-200 rpm; and
   g - washing the biological tissue with saline-phosphate buffer (PBS) comprising from 0.05-1% w/v EDTA, 1-2 times, for up to 5 minutes, at a temperature of 25-37°C, under stirring between 80-200 rpm; and
- subjecting the obtained decellularized biomaterial to a decontamination and/or sterilization process.

Moreover, the stirring in steps (a)-(g) ranges from 90-190 rpm. Said isotonic buffer of steps (a) and (e) is a buffered TRIS saline solution (TBS)comprising 10-100 mM TRIS and 100-200 mM NaCl. The hypotonic buffer of step (b)comprises from 5-20 mM of TRIS; 0.05-1% w/v of EDTA and pH between 6-8. The detergent buffer of step (c) comprises from 5-20 mM TRIS; 0.05-1% w/v of EDTA and 0.05-2% w/v SDS. Nuclease buffer from step (f) comprises 10-50 mM TRIS; 2-10 mM MgCl₂; 1-80 U/mL DNAse; and/or 0.05-80 U/mL RNAse. Step (d) comprises at least one wash water exchange in half the total time. This method can be performed in a semi-automated way for large-scale production, and preferably steps (a)-(g) can be performed in a bioreactor.

The decontamination step of the decellularized biomaterial comprises at least the use of one antibiotic and/or antimycotics.

The decontamination step of the decellularized biomaterial using antibiotics and/or antimycotics comprises:
i - washing the decellularized biomaterial at least once with sterile saline-phosphate buffer (PBS);
ii - soaking the decellularized biomaterial in sterile saline-phosphate buffer (PBS) containing concentrated solution of antibiotic and/or antimycotic;
iii - maintaining the decellularized biomaterial soaked in that solution for about 36-60 hours at a temperature of 2-8°C; and
iv - washing the decellularized and sterilized biomaterial at least once with sterile saline-phosphate buffer (PBS) .

The concentrated solution of antibiotic and/or antimycotic comprises at least one compound with antibiotic activity and at least one compound with antimycotic activity.

Preferably, the concentrated solution of antibiotic and/or antimycotic comprises: penicillin, streptomycin and amphotericin B.

The sterilization step of the decellularized biomaterial comprises the use of ethylene oxide or any other method known by one person skilled in the art, for example, gamma radiation.

Said method of producing decellularized biomaterial further comprises a dehydration step that may occur before or after the decontamination and/or sterilization step.

The dehydration step comprises subjecting the decellularized biomaterial or the decontaminated and decellularized biomaterial to a dehydration process in which said biomaterial is incubated with increasing concentrations of an alcohol, preferably wherein the alcohol concentration increases by 45%, 55%, 65%, 75%, 85%, 95% up to 100% volume/volume, and wherein said biomaterial is incubated for at least 3 hours in each of the so-called alcohol concentrations.

The method may further comprise a rehydration step of the dehydrated biomaterial, which can occur preferably after the sterilization step, wherein it comprises incubating said dehydrated biomaterial for about 20-40 minutes, with at least one substance selected from the group that consists of: water, alcohol, water or alcohol soluble substances, nutrients, metabolites, growth factors, signaling molecules, regulatory molecules, hormone, medicaments and/or pharmaceutically active compounds (e.g.: simvastatin).

The application also describes the decellularized biomaterial produced by this method, wherein it comprises a biological decellularized extracellular matrix, wherein said matrix predominantly comprises types I and III collagen fibers and elastin. The biomaterial may be used in combination with at least one active substance or it may comprise at least one active substances selected from the group that consists of: water or alcohol soluble substances, nutrients, metabolites, growth factors, signaling molecules, regulatory molecules, hormones, medicines and/or pharmaceutically active compounds (e.g.: simvastatin).

The biomaterial is for use as or for production of a scaffold product, prosthesis or carrier of active substances for systemic or localized release in an individual in need thereof. For example, there is no limitation for its use in processes of tissue repair and/or regeneration, preferably of bone tissue, cartilaginous tissue, joint, tendon, skin, for prosthetic purposes in implant dentistry and/or bucomaxillofacial traumatology, for surgical procedures of filling and/or anatomic repair, or in the production of prosthesis.

Although this patent application described the subject matter of this invention with a certain degree of detailing, by way of illustration and example for the purposes of clarity and understanding, it will be evident that certain changes and modifications may be implemented in the scope of the attached claims.

The examples described in specification are not limiting, allowing one person skilled in the art to change some aspects or components of this invention, without standing apart from the scope of the present invention.

### References

Abbas, A. K., Lichtman, A. H., & Pillai, S. (2007). Cellular and molecular immunology (6th ed.).
Badylak, S. F. (2002). The extracellular matrix as a scaffold for tissue reconstruction. Seminars in Cell & Developmental Biology, 13(5), 377-383. Retrieved from http://www.ncbi.nlm.nih.gov/pubmed/12324220
Badylak, S. F., & Gilbert, T. W. (2008). Immune response to biologic scaffold materials. Seminars in Immunology, 20(2), 109-116. https://doi.org/10.1016/j.smim. 2007.11.003
Barbanti, S. H., Zavaglia, C. A. C., & Duek, E. A. R. (2005). Polímeros bioreabsorvíveis na engenharia de tecidos. Polímeros, 15(1), 13-21. https://doi.org/10.1590 /S0104-14282005000100006
Dahl, S. L. M., Koh, J., Prabhakar, V., & Niklason, L. E. (2003). Decellularized Native and Engineered Arterial Scaffolds for Transplantation, 12(919), 659-666.
Fu, Y., Fan, X., Tian, C., Luo, J., Zhang, Y., Deng, L., ... Lv, Q. (2016). Decellularization of porcine skeletal muscle extracellular matrix for the formulation of a matrix hydrogel: a preliminary study. Journal of Cellular and Molecular Medicine, 20(4), 740-749. https://doi.org/10.1111/jcmm.12776
Gilpin, A., & Yang, Y. (2017). Decellularization Strategies for Regenerative Medicine: From Processing Techniques to Applications. BioMed Research International, 2017, 1-13. https://doi.org/10.1155/2017/9831534
Liu, Y., Bharadwaj, S., Lee, S. J., Atala, A., & Zhang, Y. (2009). Biomaterials Optimization of a natural collagen scaffold to aid cell - matrix penetration for urologic tissue engineering. Biomaterials, 30(23-24), 3865-3873. https://doi.org/10.1016/j.biomaterials.2009.04.008
Martinello, T., Bronzini, I., Volpin, A., Vindigni, V., Maccatrozzo, L., Caporale, G., ... Patruno, M. (2014). Successful recellularization of human tendon scaffolds using adipose-derived mesenchymal stem cells and collagen gel. Journal of Tissue Engineering and Regenerative Medicine, 8(8), 612-619. https://doi.org/10.1002/term.1557
Meng, F., Modo, M., & Badylak, S. F. (2014). Biologic scaffold for CNS repair. Regenerative Medicine, 9(3), 367-383. https://doi.org/10.2217/rme.14.9
Mohammadie, Z. M., Parivar, K., Shahri, N. M., Fereidoni, M., & Hayati-Roodbari, N. (2018). Decellularized Bovine Articular Cartilage Matrix Reinforced by Carboxylated-SWCNT for Tissue Engineering Application. Brazilian Archives of Biology and Technology, 60. https://doi.org/10.1590/1678-4324-2017160083
Paduano, F., Marrelli, M., White, L. J., Shakesheff, K. M., & Tatullo, M. (2016). Odontogenic Differentiation of Human Dental Pulp Stem Cells on Hydrogel Scaffolds Derived from Decellularized Bone Extracellular Matrix and Collagen Type I. PLOS ONE, 11(2), e0148225. https://doi.org/10.1371/journal.pone.0148225
Parenteau-Bareil, R., Gauvin, R., & Berthod, F. (2010). Collagen-Based Biomaterials for Tissue Engineering Applications. Materials, 3(3), 1863-1887. https://doi.org/10.3390/ma3031863
Sackett, S. D., Tremmel, D. M., Ma, F., Feeney, A. K., Maguire, R. M., Brown, M. E., ... Odorico, J. S. (2018). Extracellular matrix scaffold and hydrogel derived from decellularized and delipidized human pancreas. Scientific Reports, 8(1), 10452. https://doi.org/10.1038/s41598-018-28857-
Saldin, L. T., Cramer, M. C., Velankar, S. S., White, L. J., & Badylak, S. F. (2017). Extracellular matrix hydrogels from decellularized tissues: Structure and function. Acta Biomaterialia, 49, 1-15. https://doi.org/10.1016/j.actbio.2016.11.068
Sawkins, M. J., Bowen, W., Dhadda, P., Markides, H., Sidney, L. E., Taylor, A. J., ... White, L. J. (2013). Hydrogels derived from demineralized and decellularized bone extracellular matrix. Acta Biomaterialia, 9(8), 7865-7873. https://doi.org/10.1016/j.actbio.2013.04.029
Seif-naraghi, S. B., Horn, D., Schup-magoffin, P. J., & Christman, K. L. (2012). Acta Biomaterialia Injectable extracellular matrix derived hydrogel provides a platform for enhanced retention and delivery of a heparin-binding growth factor. Acta Biomaterialia, 8(10), 3695-3703. https://doi.org/10.1016/j.actbio.2012.06.030
Singelyn, J. M., & Christman, K. L. (n.d.). Modulation of Material Properties of a Decellularized Myocardial Matrix Scaffold, 731-738. https://doi.org/10.1002/ mabi.201000423
Tedder, M. E., Liao, J., Ph, D., Weed, B., Stabler, C., Zhang, H., ... Ph, D. (2009). Stabilized Collagen Scaffolds for Heart Valve Tissue Engineering, 15(6).
Ungerleider, J. L., Johnson, T. D., Rao, N., & Christman, K. L. (2015). Fabrication and characterization of injectable hydrogels derived from decellularized skeletal and cardiac muscle. Methods, 84, 53-59. https://doi.org/10.1016/j.ymeth.2015.03.024
Visser, J. (n.d.). Crosslinkable Hydrogels derived from Cartilage , Meniscus and Tendon Tissue, 1-34.
Wang, B., Borazjani, A., Tahai, M., de Jongh Curry, A. L., Simionescu, D. T., Guan, J., ... Liao, J. (2010). Fabrication of cardiac patch with decellularized porcine myocardial scaffold and bone marrow mononuclear cells. Journal of Biomedical Materials Research Part A, 9999A, NA-NA. https://doi.org/10.1002/jbm.a.32781
Wang, Y., Bao, J., Wu, Q., Zhou, Y., Li, Y., Wu, X., ... Bu, H. (2015). Method for perfusion decellularization of porcine whole liver and kidney for use as a scaffold for clinical-scale bioengineering engrafts. Xenotransplantation, 22(1), 48-61. https://doi.org/10.1111 /xen.12141
Wolf, M. T., Daly, K. A., Brennan-Pierce, E. P., Johnson, S. A., Carruthers, C. A., D'Amore, A., ... Badylak, S. F. (2012). A hydrogel derived from decellularized dermal extracellular matrix. Biomaterials, 33(29), 7028-7038. https://doi.org/10.1016/j.biomaterials.2012.06.051
Wu, J., Ding, Q., Dutta, A., Wang, Y., Huang, Y., Weng, H., ... Hong, Y. (2015). An injectable extracellular matrix derived hydrogel for meniscus repair and regeneration. Acta Biomaterialia, 16, 49-59. https://doi.org/10.1016/j.actbio.2015.01.027
Xu, K., Kuntz, L. A., Foehr, P., Kuempel, K., Wagner, A., Tuebel, J., ... Burgkart, R. H. (2017). Efficient decellularization for tissue engineering of the tendon-bone interface with preservation of biomechanics. PLOS ONE, 12(2), e0171577. https://doi.org/10.1371/journal. pone.0171577

## Claims

1. A method of producing decellularized biomaterial, comprising the steps of:
- collecting, processing and cleaning biological tissue;
- subjecting the biological tissue to a decellularization process, comprising:
a- washing the biological tissue 1-5 times using isotonic buffer between 1-20 minutes, at a temperature of 0-25°C, under stirring between 80-200 rpm;
b- incubating the biological tissue using hypotonic buffer for 1-18 hours at a temperature between 4-37°C under stirring between 80-200 rpm;
c- treating the biological tissue with detergent buffer, for 5-24 hours, at a temperature between 4-37°C under stirring between 80 and 200 rpm;
d- washing the biological tissue with deionized water for 30 minutes to 4 hours, at a temperature between 4-37°C under stirring between 80-200 rpm;
e- washing the biological tissue 1-5 times with isotonic buffer, for 5-15 minutes, at a temperature between 4-37°C under stirring between 80-200 rpm;
f- incubating the biological tissue with nuclease buffer, between 30 minutes-3 hours, at a temperature of 30-40°C, under stirring between 80-200 rpm; and
g- washing the biological tissue with saline-phosphate buffer (PBS) with chelating agent, 1-2 times, for up to 5 minutes, at a temperature of 25-37°C, under stirring between 80-200 rpm; and
- subjecting the decellularized biomaterial to a decontamination and/or sterilization process.

2. The method of claim 1, wherein said biological tissue is derived from eye's connective tissue, preferably sclera of porcine, bovine, rabbit or human origin.

3. The method of claim 1, wherein the stirring in steps (a)-(g) ranges from 90-190 rpm.

4. The method of claim 1, wherein said isotonic buffer of steps (a) and (e) is a buffered TRIS saline solution (TBS) comprised of 10-100 mM TRIS and 100-200 mM NaCl.

5. The method of claim 1, wherein the hypotonic buffer of step (b) comprises from 5-20 mM of TRIS; 0.05-1% w/v of EDTA and pH between 6-8.

6. The method of claim 1, wherein the detergent buffer of step (c) comprises from 5-20 mM of TRIS; 0.05-1% w/v of EDTA and 0.05-2% w/v SDS.

7. The method of claim 1, wherein the nuclease buffer of step (f) comprises from 10-50 mM TRIS; 2-10 mM MgCl₂; 1-80 U/mL DNAse; and/or 0.05-80 U/mL RNAse.

8. The method of claim 1, wherein the step (d) comprises at least one wash water exchange in half the total time.

9. The method of any one of claims 1-8, wherein said method can be performed in a semi-automated way for large-scale production, and preferably steps (a)-(g) can be performed in a bioreactor.

10. The method of any one of claims 1-9, wherein the decontamination step of the decellularized biomaterial comprises the use of at least one antibiotics and/or antimycotics.

11. The method of claim 10, wherein the step of decontaminating the decellularized biomaterial using at least one antibiotics and/or antimycotics comprises:
i - washing the decellularized biomaterial at least once with sterile saline-phosphate buffer (PBS);
ii - soaking the decellularized biomaterial in sterile saline-phosphate buffer (PBS) containing concentrated solution of antibiotics and/or antimycotics;
iii - maintaining the decellularized biomaterial soaked in that solution for about 36-60 hours at a temperature of 2-8°C; and
iv - washing the decellularized and sterilized biomaterial at least once with sterile saline-phosphate buffer (PBS).

12. The method of claim 10 or 11, wherein the concentrated solution of antibiotic and/or antimycotic comprises at least one compound with antibiotic action and at least one compound with antimycotic action.

13. The method of claim 1-12, wherein the sterilization step of the decellularized biomaterial comprises the use of ethylene oxide or gamma radiation.

14. The method of claim 1-13, wherein said method further comprises a dehydration step that can occur preferably after a decontamination step and before the sterilization step.

15. The method of claim 14, wherein the dehydration step comprises subjecting the decellularized biomaterial or the decellularized and decontaminated biomaterial to a dehydration process in which said biomaterial is incubated with increasing concentrations of an alcohol, preferably wherein the alcohol concentration increases by 45%, 55%, 65%, 75%, 85%, 95% up to 100% volume/volume, and wherein said biomaterial is incubated for at least 3 hours in each of the so-called alcohol concentrations.

16. The method of any one of claims 14 or 15, wherein the method may further comprise a rehydration step of the dehydrated biomaterial, wherein it comprises incubating said dehydrated biomaterial for about 20-40 minutes, with at least one substance selected from the group that consists of: water, alcohol, water or alcohol soluble substances, nutrients, metabolites, growth factors, signaling molecules, regulatory molecules, hormone, medicaments and/or pharmaceutically active compounds.

17. A decellularized biomaterial wherein it is produced by the method as defined in any one of claims 1 to 16 wherein it comprises a decellularized extracellular matrix of biological origin, wherein said matrix predominantly comprises collagen type I, III and elastin fibers.

18. Biomaterial of claim 17, wherein it further comprises active substances selected from the group that consists of: water or alcohol soluble substances, nutrients, metabolites, growth factors, signaling molecules, regulatory molecules, hormones, medicaments and/or pharmaceutically active compounds.

19. Biomaterial of any one of claims 17 or 18, wherein it is for use as a scaffold, prosthesis or carrier of active substances in an individual in need thereof.

20. The decellularized biomaterial as claim 19, wherein it is for use in tissue repair and/or regeneration processes, preferably bone tissue, cartilaginous tissue, joints, tendons, skin, for prosthetic purposes in implantology and/or oral and maxillofacial traumatology, for surgical filling and /or anatomical correction or in the production of prostheses.

21. Use of the biomaterial as produced by the method of any one of claims 1 or 16, wherein it is for use in the production of a scaffold, in the production of prosthesis and/or in the production of a carrier of substances for systemic or localized release in an individual in need thereof.

22. Use of claim 20, wherein it is for use in processes of tissue repair and/or regeneration processes, preferably bone tissue, cartilaginous tissue, joints, tendons, skin, for prosthetic purposes in implantology and/or oral and maxillofacial traumatology, for surgical filling and /or anatomical correction or in the production of prostheses.
